# EUROPEAN PATENT SPECIFICATION

(11) **EP 3 741 845 B1**
(45) Date of publication and mention of the grant of the patent: **18.06.2025**
(21) Application number: 19741656.3
(22) Date of filing: 18.01.2019
(51) Int. Cl.: C12N 7/00, C12N 9/26, C07K 14/47, C12N 15/86, C07K 14/54, C07K 14/705, A61K 35/768, A61P 35/00

(54) **RECOMBINANT VACCINIA VIRUS AND PHARMACEUTICAL COMPOSITION COMPRISING SAME**
REKOMBINANTES VACCINIA-VIRUS UND PHARMAZEUTISCHE ZUSAMMENSETZUNG DAMIT
VIRUS DE LA VACCINE RECOMBINANT ET COMPOSITION PHARMACEUTIQUE LE COMPRENANT

(30) Priority: 19.01.2018 KR 20180007381
(43) Date of publication of application: 25.11.2020
(73) Proprietor: Kolon Life Science, Inc., Seoul 07793 (KR)
(72) Inventor: KIM, Sujeong, Seoul 07062 (KR); CHOI, Heonsik, Seoul 07030 (KR); KIM, Minjung, Seoul 02034 (KR); SHIN, Jaeil, Seoul 04731 (KR); HONG, Soonoh, Yongin-si, Gyeonggi-do 16846 (KR); LEE, Hyesun, Seoul 06088 (KR); LEE, Soondong, Gwangmyeong-si, Gyeonggi-do 14247 (KR); CHOI, Hwanjun, Seoul 07041 (KR); KIM, Joonsung, Suwon-si, Gyeonggi-do 16418 (KR); HONG, Jieun, Gwacheon-si, Gyeonggi-do 13837 (KR); LEE, Eunjin, Seoul 08521 (KR); KANG, Haesu, Seoul 06762 (KR)
(74) Representative: HGF
(86) International application number: PCT/KR2019/000797
(87) International publication number: WO 2019/143201

(56) References cited:
- EP-A1- 3 305 904
- WO-A1-2014/047350
- WO-A1-2016/008976
- WO-A1-2016/033555
- WO-A1-2016/195332
- WO-A1-2017/044780
- WO-A1-2018/057755
- KR-A- 20170 003 920
- ZUQIANG LIU ET AL: "Rational combination of oncolytic vaccinia virus and PD-L1 blockade works synergistically to enhance therapeutic efficacy", NATURE COMMUNICATIONS, vol. 8, 27 March 2017 (2017-03-27), pages 14754, XP055452350, DOI: 10.1038/ncomms14754
- MEE Y. BARTEE ET AL: "Tumor-Localized Secretion of Soluble PD1 Enhances Oncolytic Virotherapy", CANCER RESEARCH, vol. 77, no. 11, 1 June 2017 (2017-06-01), US, pages 2952 - 2963, XP055515259, ISSN: 0008-5472, DOI: 10.1158/0008-5472.CAN-16-1638
- CHEN CHUN-YU ET AL: "Oncolytic virus and PD-1/PD-L1 blockade combination therapy", ONCOLYTIC VIROTHERAPY, vol. Volume 7, 31 July 2018 (2018-07-31), pages 65 - 77, XP055796308, Retrieved from the Internet <URL:https://www.dovepress.com/getfile.php?fileID=43409> DOI: 10.2147/OV.S145532
- SONIA GUEDAN ET AL: "Hyaluronidase Expression by an Oncolytic Adenovirus Enhances Its Intratumoral Spread and Suppresses Tumor Growth", MOLECULAR THERAPY, vol. 18, no. 7, 4 May 2010 (2010-05-04), pages 1275 - 1283, XP055010308, ISSN: 1525-0016, DOI: 10.1038/mt.2010.79
- FROST GREGORY I: "Recombinant human hyaluronidase (rHuPH20): an enabling platform for subcutaneous drug and fluid administration", EXPERT OPINION ON DRUG DELIVERY, INFORMA HEALTHCARE, GB, vol. 4, no. 4, 1 July 2007 (2007-07-01), pages 427 - 440, XP008177677, ISSN: 1742-5247, [retrieved on 20070807], DOI: 10.1517/17425247.4.4.427
- VEINALDE RUTA ET AL: "Oncolytic measles virus encoding interleukin-12 mediates potent antitumor effects through T cell activation", ONCOIMMUNOLOGY, vol. 6, no. 4, 3 April 2017 (2017-04-03), pages e1285992, XP055820999, Retrieved from the Internet <URL:https://www.tandfonline.com/doi/pdf/10.1080/2162402X.2017.1285992?needAccess=true> DOI: 10.1080/2162402X.2017.1285992
- GMACHL, M.: "The human sperm protein PH -20 has hyaluronidase activity", FEBS LETT., vol. 336, 28 December 1993 (1993-12-28), pages 545 - 548, XP025607670, doi:10.1016/0014-5793(93)80873-S
- ZHOU, J.: "PD1-based DNA vaccine amplifies HIV-1 GAG-specific CD 8+ T cells in mice", J. CLIN. INVEST., vol. 123, 1 May 2013 (2013-05-01), pages 2629 - 2642, XP055274840, doi:10.1172/JCI64704

## Description

### Technical Field

The present invention relates to a recombinant vaccinia virus comprising a gene encoding soluble programmed cell death protein-1 (sPD-1), a gene encoding hyaluronidase, and a gene encoding interleukin (IL)-12 , and a pharmaceutical composition comprising same.

### Background Art

Recently, studies on oncolytic viruses modified by genetically manipulating various viruses have been actively conducted for the purpose of developing cancer therapeutic agents. However, problems with the oncolytic viruses have yet to be fully resolved. For example, in order to be developed into an anticancer agent, a virus having tumor-selective replication ability was produced through genetic manipulation. However, the following problems have been found: such a virus kills normal cells as well as cancer cells, and also has insufficient anticancer effects. Accordingly, there is a continuing demand for development of a technique which allows the oncolytic viruses to have increased selectivity and efficacy against cancer cells while minimizing influences on normal cells.

On the other hand, vaccinia virus is an enveloped DNA virus with doublestranded linear genomic DNA of about 200 kbp which encodes about 200 independent genes. The vaccinia virus was first used by Edward Jenner in the eighteenth century as a prophylactic vaccine for smallpox. Since then, the vaccinia virus has been developed into various prophylactic vaccines. In early vaccinia virus vaccines, a wild-type virus was used, and vaccinated patients showed serious side effects such as systemic infection or progressive infection. Therefore, in order to reduce side effects, modified vaccinia viruses with attenuated toxicity such as modified vaccinia Ankara (MVA), LC16m8 (derived from the Lister strain), and New York vaccinia virus (NYVAC, derived from the Copenhagen vaccinia strain) were developed. Vaccines that are applicable to various diseases have been developed based on these vaccinia viruses. In particular, vaccinia virus strains such as Western Reserve (WR), NYVAC, Wyeth, and Lister are also being developed as oncolytic viruses.
WO2016/008976 discloses a vaccinia virus expressing a checkpoint inhibitor. WO2014/047350 discloses a vaccinia virus expressing a compound antagonizing the activity of PD-1 and expressing IL-12.
Zuqiang Liu et al. teaches the combination of a oncolytic vaccinia virus and a checkpoint inhibitor for use in the treatment of cancer.
Mee Y. Bartee et al. discloses an oncolytic myxomavirus virus and a checkpoint inhibitor for use in the treatment of cancer.
WO2018/057755 discloses oncolytic vaccinia viruses expressing a hyaluronidase.

### Disclosure of Invention

### Technical Problem

An object of the present invention is to provide a recombinant vaccinia virus comprising a gene encoding soluble programmed cell death protein-1 (sPD-1), a gene encoding hyaluronidase, and a gene encoding interleukin (IL)-12and optionally having suppressed expression of some genes therein, and a pharmaceutical composition for use in preventing or treating cancer, comprising same as an active ingredient.

### Solution to Problem

In order to achieve the above object, the present invention provides a recombinant vaccinia virus, comprising a gene encoding soluble programmed cell death protein-1 (sPD-1), a gene encoding hyaluronidase, and a gene encoding interleukin (IL)-12.

In addition, the present invention provides a composition that contains, as an active ingredient, the recombinant vaccinia virus of the invention, for use in preventing or treating cancer.

### Advantageous Effects of Invention

The recombinant vaccinia virus according to the present invention, has an excellent anti-tumor effect. Therefore, the recombinant vaccinia virus of the present invention can be usefully used to treat cancer.

### Brief Description of Drawings

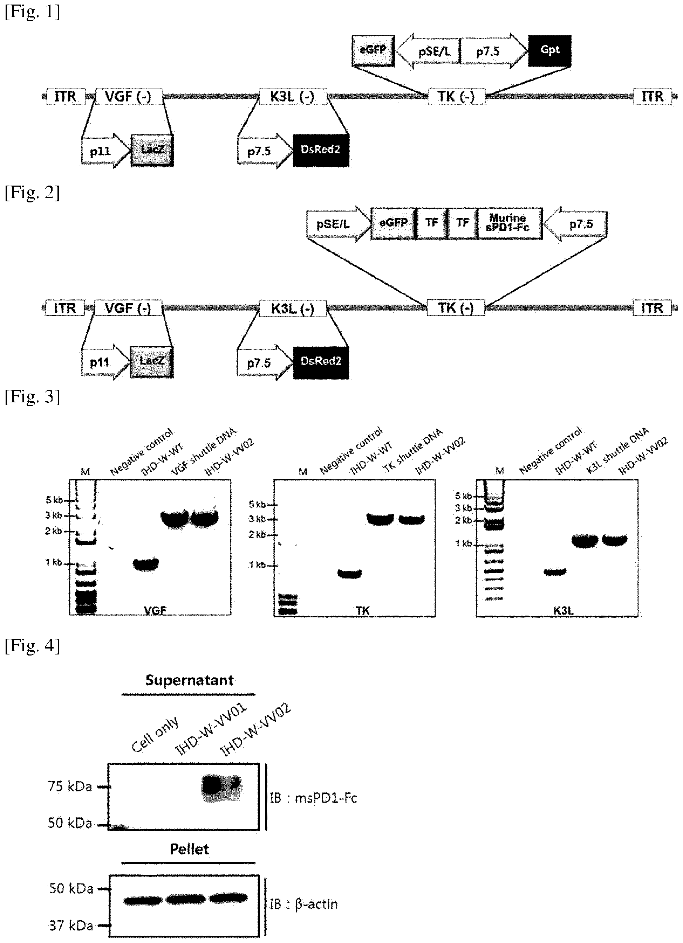

Fig. 1 illustrates a schematic diagram of the gene structure of IHD-W vaccinia virus (IHD-W-VV01) in which VGF, TK, and K3L are deleted. The abbreviated terms related to viral gene deletion and therapeutic gene introduction have the meanings as shown in Table 1 below.

**[Table 1]**

| Abbreviation | Meaning |
|---|---|
| IHD-W-VV01 | Recombinant IHD-W vaccinia virus in which VGF, TK, and K3L are deleted |
| IHD-W-VV02 | Recombinant IHD-W vaccinia virus in which VGF, TK, and K3L are deleted and expression of sPD1-Fc gene is induced |
| IHD-W-VV03 | Recombinant IHD-W vaccinia virus in which VGF, TK, and K3L are deleted and expression of PH20 gene is induced |
| IHD-W-VV04 | Recombinant IHD-W vaccinia virus in which VGF, TK, and K3L are deleted and expression of IL-12 gene is induced |
| IHD-W-VV05 | Recombinant IHD-W vaccinia virus in which VGF, TK, and K3L are deleted and expression of sPD1-Fc and PH20 genes is induced |
| IHD-W-VV06 | Recombinant IHD-W vaccinia virus in which VGF, TK, and K3L are deleted and expression of sPD1-Fc, PH20, and IL-12 genes is induced |

Fig. 2 illustrates a schematic diagram of the gene structure of IHD-W-VV02 in which VGF, TK, and K3L are deleted and into which sPD1-Fc gene is inserted.
Fig. 3 illustrates results obtained by performing electrophoresis of PCR product of IHD-W-VV02 gDNA, which identifies that the sPD1-Fc gene has been inserted.
Fig. 4 illustrates results obtained by performing Western blotting of proteins secreted from HeLa cells infected with IHD-W-VV02, which shows that the sPD1-Fc gene has been expressed.
Fig. 5 illustrates a schematic diagram of the gene structure of IHD-W-VV03 in which VGF, TK, and K3L are deleted and into which PH20 gene is inserted.
Fig. 6 illustrates results obtained by performing electrophoresis of PCR product of IHD-W-VV03 gDNA, which shows that the PH20 gene has been inserted.
Fig. 7 illustrates results obtained by performing Western blotting of proteins secreted from HeLa cells infected with IHD-W-VV03, which shows that the PH20 gene has been expressed.
Fig. 8 illustrates a schematic diagram of the gene structure of IHD-W-VV04 in which VGF, TK, and K3L are deleted and into which IL-12 gene is inserted.
Fig. 9 illustrates results obtained by performing electrophoresis of PCR product of IHD-W-VV04 gDNA, which shows that the IL-12 gene has been inserted.
Fig. 10 illustrates results obtained by performing Western blotting of proteins secreted from HeLa cells infected with IHD-W-VV04, which shows that the IL-12 gene has been expressed.
Fig. 11 illustrates a schematic diagram of the gene structure of IHD-W-VV05 in which VGF, TK, and K3L are deleted and into which sPD1-Fc and PH20 genes are inserted.
Fig. 12 illustrates results obtained by performing electrophoresis of PCR product of IHD-W-VV05 gDNA, which identifies that the sPD1-Fc and PH20 genes have been inserted.
Fig. 13 illustrates results obtained by performing Western blotting of proteins secreted from HeLa cells infected with IHD-W-VV05, which shows that the sPD1-Fc and PH20 genes have been expressed.
Fig. 14 illustrates a schematic diagram of the gene structure of IHD-W-VV06 in which VGF, TK, and K3L are deleted and into which sPD1-Fc, PH20, and IL-12 genes are inserted.
Fig. 15 illustrates results obtained by performing electrophoresis of PCR product of IHD-W-VV06 gDNA, which identifies that the sPD1-Fc, PH20, and IL-12 genes have been inserted.
Fig. 16 illustrates results obtained by performing Western blotting of proteins secreted from HeLa cells infected with IHD-W-VV06, which shows that the sPD1-Fc, PH20, and IL-12 genes have been expressed.
Fig. 17 illustrates a schematic diagram of the gene structure of WR vaccinia virus (WR-VV01) in which expression of VGF, TK, and K3L are inactivated. The abbreviated terms related to viral gene inactivation and therapeutic gene introduction have the meanings as shown in Table 2 below.

**[Table 2]**

| Abbreviation | Meaning |
|---|---|
| WR-VV01 | Recombinant WR vaccinia virus in which expression of VGF, TK, and K3L is inactivated |
| WR-VV05 | Recombinant WR vaccinia virus in which expression of VGF, TK, and K3L is inactivated and expression of sPD1-Fc and PH20 genes is induced |
| WR-VV06 | Recombinant WR vaccinia virus in which expression of VGF, TK, and K3L is inactivated and expression of sPD1-Fc, PH20, and IL-12 genes is induced |

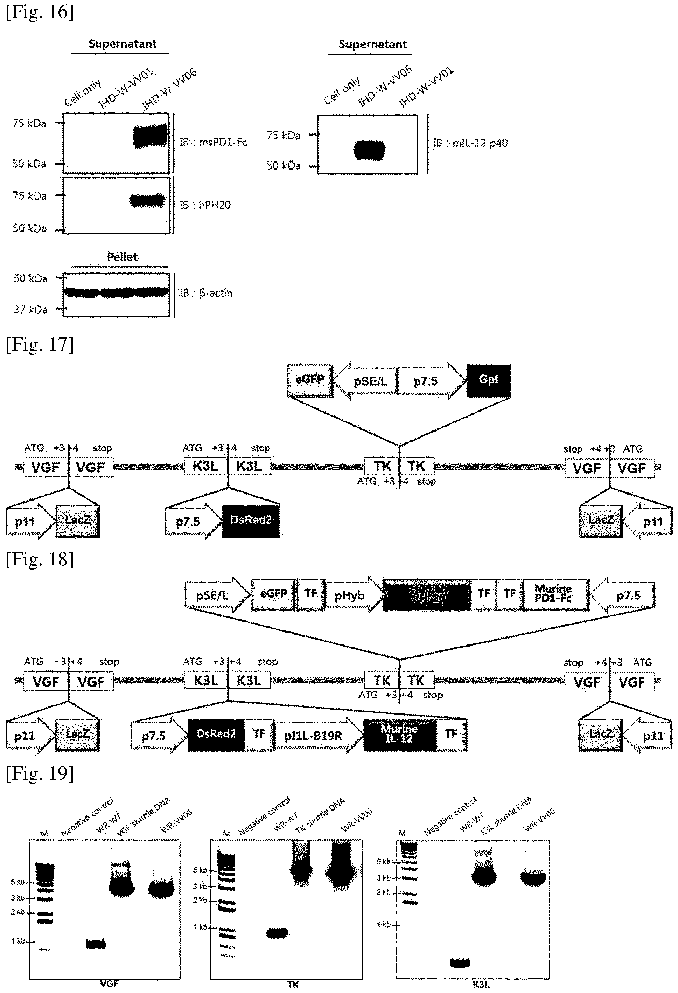

Fig. 18 illustrates a schematic diagram of the gene structure of WR-VV06 in which expression of VGF, TK, and K3L is inactivated and into which sPD1-Fc, PH20, and IL-12 genes are inserted.
Fig. 19 illustrates results obtained by performing electrophoresis of PCR product of WR-VV06 gDNA, which shows that the sPD1-Fc, PH20, and IL-12 genes have been inserted.

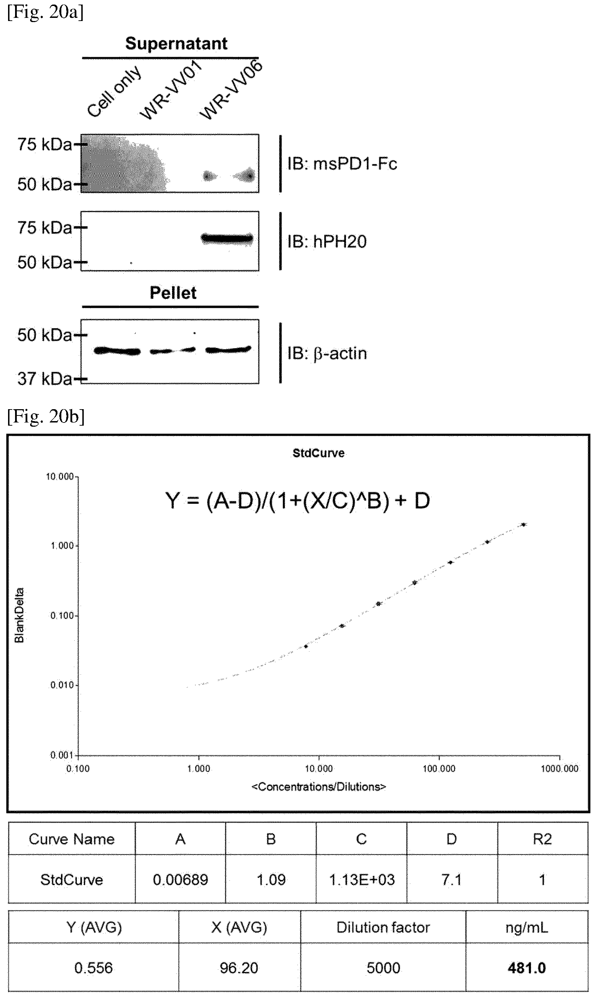

Fig. 20a illustrates results obtained by performing Western blotting of proteins secreted from HeLa cells infected with WR-VV06, which shows that the sPD1-Fc, PH20 genes have been expressed.
Fig. 20b illustrates results obtained by performing ELISA on proteins secreted from HeLa cells infected with WR-VV06, which shows that the IL-12 gene has been expressed.

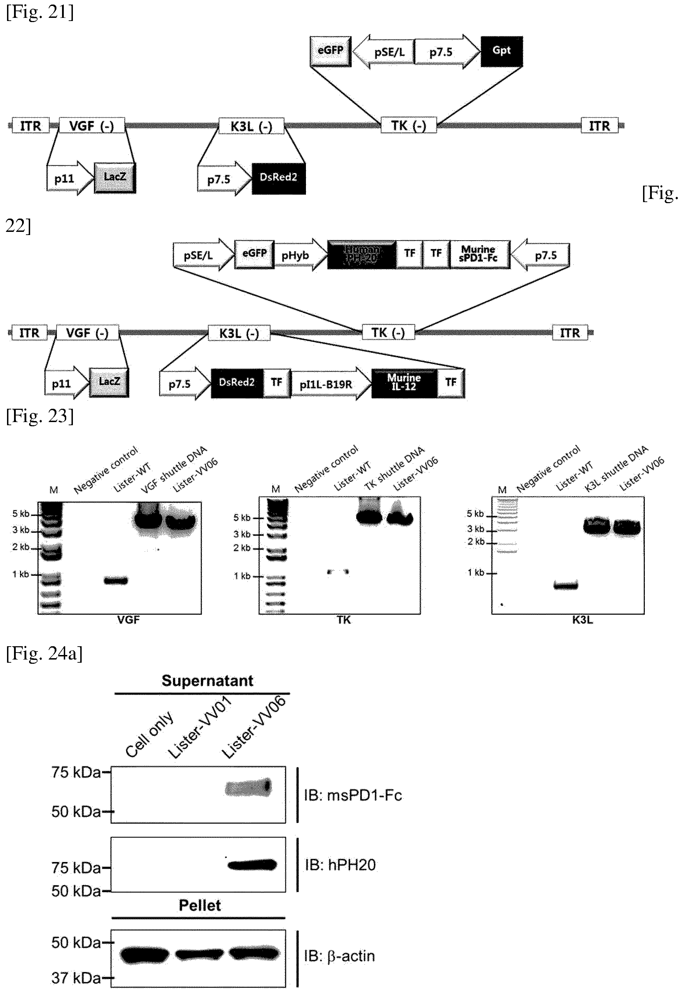

Fig. 21 illustrates a schematic diagram of the gene structure of Lister vaccinia virus (Lister-VV01) in which VGF, TK, and K3L are deleted. The abbreviated terms related to viral gene deletion and therapeutic gene introduction have the meanings as shown in Table 3 below.

**[Table 3]**

| Abbreviation | Meaning |
|---|---|
| Lister-VV01 | Recombinant Lister vaccinia virus in which VGF, TK, and K3L are deleted |
| Lister-VV05 | Recombinant Lister vaccinia virus in which VGF, TK, and K3L are deleted and expression of sPD1-Fc and PH20 genes is induced |
| Lister-VV06 | Recombinant Lister vaccinia virus in which VGF, TK, and K3L are deleted and expression of sPD1-Fc, PH20, and IL-12 genes is induced |

Fig. 22 illustrates a schematic diagram of the gene structure of Lister-VV06 in which VGF, TK, and K3L are deleted and into which sPD1-Fc, PH20, and IL-12 genes are inserted.
Fig. 23 illustrates results obtained by performing electrophoresis of PCR product of Lister-VV06 gDNA, which shows that the sPD1-Fc, PH20, and IL-12 genes have been inserted.
Fig. 24a illustrates results obtained by performing Western blotting of proteins secreted from HeLa cells infected with Lister-VV06, which shows that the sPD1-Fc, PH20 genes have been expressed.

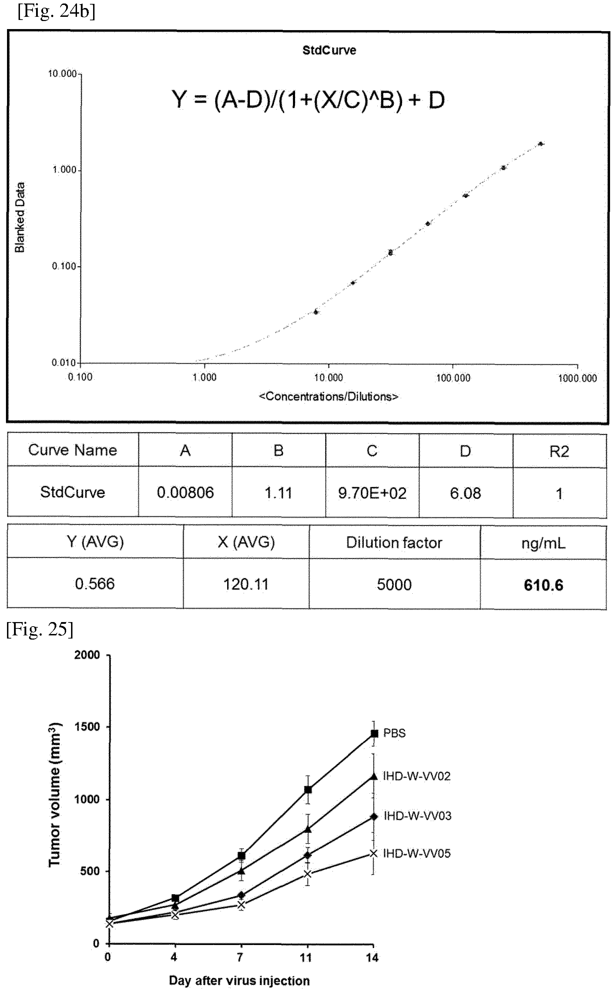

Fig. 24b illustrates results obtained by performing ELISA on proteins secreted from HeLa cells infected with Lister-VV06, which shows that the IL-12 gene has been expressed.
Fig. 25 illustrates results obtained by intratumorally administering, to a mouse colorectal tumor model, IHD-W-VV02, IHD-W-VV03, and IHD-W-VV05, and then measuring tumor growth.

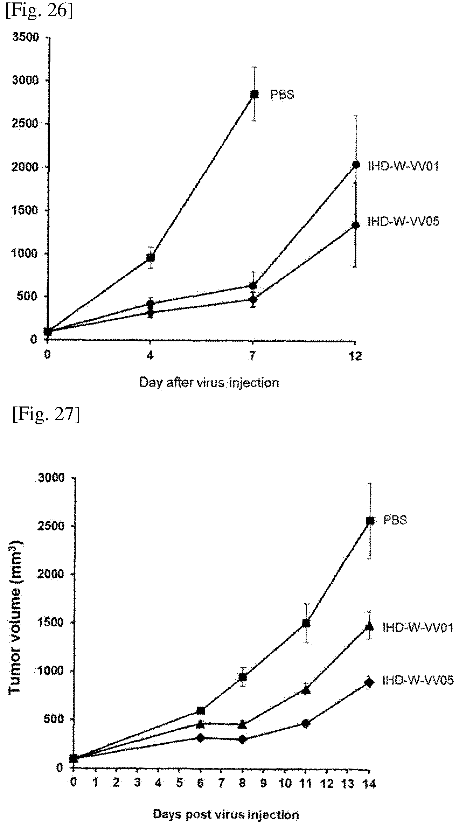

Fig. 26 illustrates results obtained by intratumorally administering, to a mouse melanoma model, IHD-W-VV01 and IHD-W-VV05, and then measuring tumor growth.
Fig. 27 illustrates results obtained by intratumorally administering, to a mouse lung tumor model, IHD-W-VV01 and IHD-W-VV05, and then measuring tumor growth.

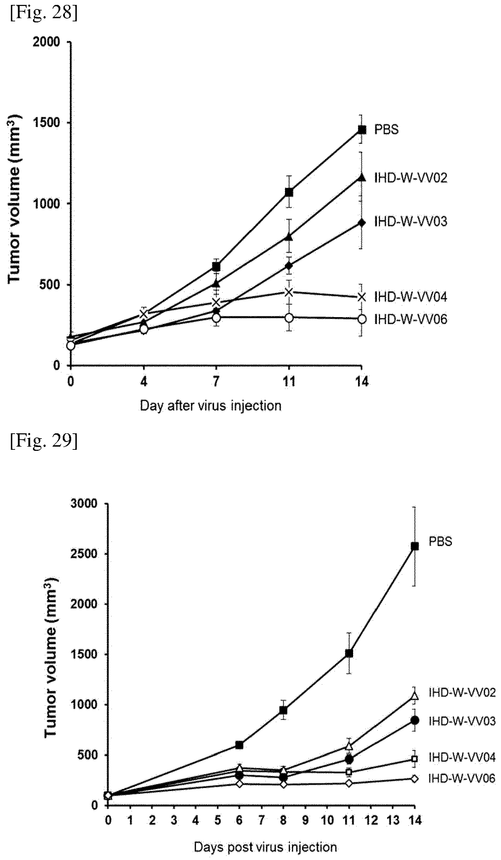

Fig. 28 illustrates results obtained by intratumorally administering, to a mouse colorectal tumor model, IHD-W-VV02, IHD-W-VV03, IHD-W-VV04, and IHD-W-VV06, and then measuring tumor growth.
Fig. 29 illustrates results obtained by intratumorally administering, to a mouse lung tumor model, IHD-W-VV02, IHD-W-VV03, IHD-W-VV04, and IHD-W-VV06, and then measuring tumor growth.

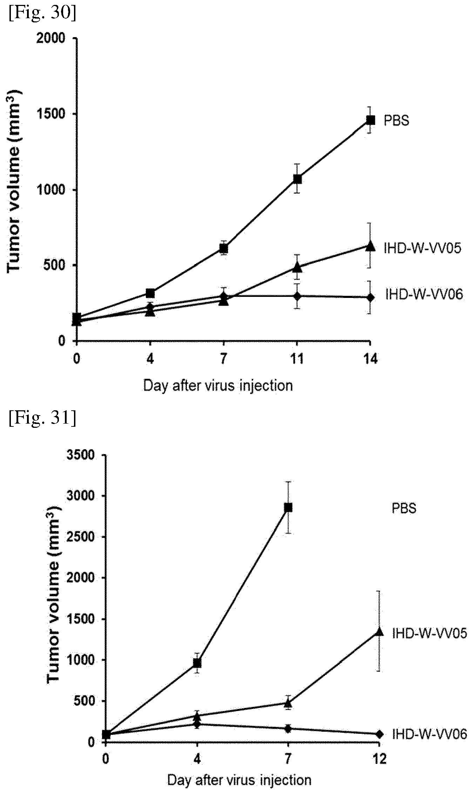

Fig. 30 illustrates results obtained by intratumorally administering, to a mouse colorectal tumor model, IHD-W-VV05 and IHD-W-VV06, and then measuring tumor growth.
Fig. 31 illustrates results obtained by intratumorally administering, to a mouse melanoma model, IHD-W-VV05 and IHD-W-VV06, and then measuring tumor growth.

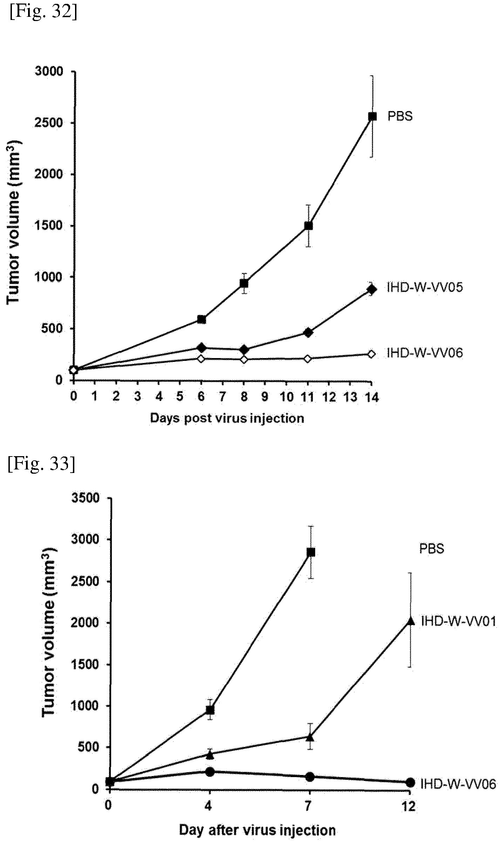

Fig. 32 illustrates results obtained by intratumorally administering, to a mouse lung tumor model, IHD-W-VV05 and IHD-W-VV06, and then measuring tumor growth.
Fig. 33 illustrates results obtained by intratumorally administering, to a mouse melanoma model, IHD-W-VV01 and IHD-W-VV06, and then measuring tumor growth.

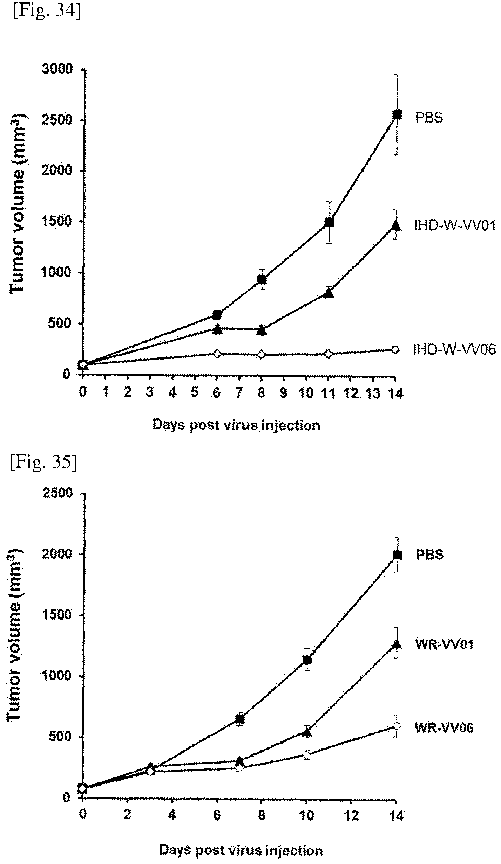

Fig. 34 illustrates results obtained by intratumorally administering, to a mouse lung tumor model, IHD-W-VV01 and IHD-W-VV06, and then measuring tumor growth.
Fig. 35 illustrates results obtained by intratumorally administering, to a mouse lung tumor model, WR-VV01 and WR-VV06, and then measuring tumor growth.

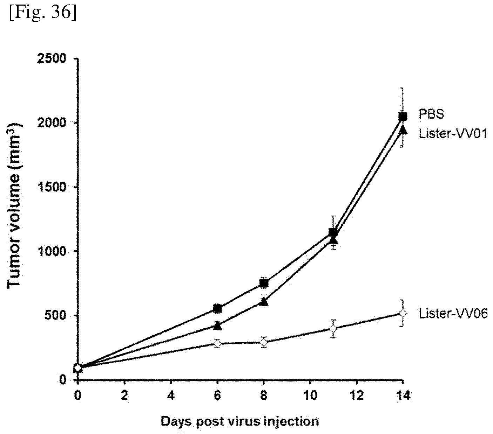

Fig. 36 illustrates results obtained by intratumorally administering, to a mouse lung tumor model, Lister-VV01 and Lister-VV06, and then measuring tumor growth.

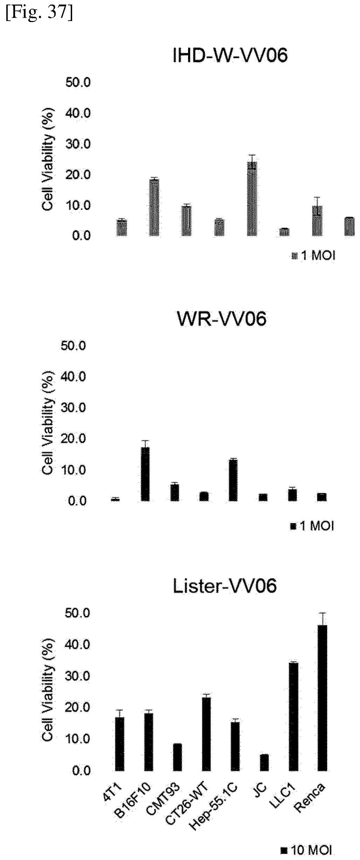

Fig. 37 illustrates results obtained by determining *in vitro* cell-killing ability of IHD-W-VV06, WR-VV06, and Lister-VV06.

### Best Mode for Carrying out the Invention

In an aspect of the present invention, there is provided a recombinant vaccinia virus comprising a gene encoding soluble programmed cell death protein-1 (sPD-1), a gene encoding hyaluronidase, and a gene encoding IL-12.

The term "sPD-1" as used herein refers to an extracellular domain or a fragment of programmed death-1 (PD-1), which is a 55-kDa type I transmembrane protein and belongs to a family of immunoglobulin molecules and is well known as a co-inhibitory molecule on T cells. In addition, the term sPD-1 may be used as having a meaning that includes sPD-1 fusion protein in which the full-length sPD-1 or a fragment of sPD-1 and an immunoglobulin Fc region are fused. A specific example of the fusion protein may be sPD-1-Fc in which sPD-1 is bound to an immunoglobulin Fc region. Here, the sPD-1 and the Fc may be bound to each other directly or via a linker. Here, the linker may be a peptide composed of 1 to 50, 2 to 45, 3 to 40, 5 to 30, or 7 to 25 amino acids, and may specifically be a peptide composed of 3 to 40 amino acids. The linker may be an amino acid sequence selected from, but is not limited to, the group consisting of GGGGS (SEQ ID NO: 123), GGGGSGGGGSGGGGSEPKSCDKTHTCPPCP (SEQ ID NO: 124), SPKAQAGGGGSAQPQAEGSLGGGGSAKASAPAGGGGS (SEQ ID NO: 125), GGSGGSGGSGGSGGSEQEER (SEQ ID NO: 126), GGGGSGGGGSGGS (SEQ ID NO: 127), SGGGGSGGGGSGGGGSGTHTCPPCP (SEQ ID NO: 128), GGSGGGGS (SEQ ID NO: 129), and GGGGSGGGGSGGGS (SEQ ID NO: 130).

The sPD-1 may be part of a sequence of GenBank: NM_008798.2, and is not limited to any one sequence. Specifically, the sPD-1 may be the amino acid sequence of SEQ ID NO: 131 or SEQ ID NO: 133. In addition, the sPD-1 may have a homology of about 90%, 91%, 92%, 93%, or 94% or more, preferably about 95%, 96%, 97%, 98%, or 99% or more, and most preferably about 99% or more, to the amino acid sequence of SEQ ID NO: 131, as long as the function of the sPD-1 is not modified.

In addition, the term "sPD-1 gene" as used herein refers to a gene encoding "sPD-1 protein" or a gene encoding "sPD-1 fusion protein" in which the sPD-1 protein, a target protein, and other proteins are fused. Specifically, the sPD-1 gene may encode sPD-1 fusion protein containing an Fc region (sPD1-Fc). The sPD-1 gene may comprise the nucleotide sequence of SEQ ID NO: 132 or 134. In addition, the sPD-1 gene may have a homology of about 90%, 91%, 92%, 93%, or 94% or more, preferably about 95%, 96%, 97%, 98%, or 99% or more, and most preferably about 99% or more, to the nucleotide sequence of SEQ ID NO: 134, as long as the function of the sPD-1 is not modified.

In addition, the term "hyaluronidase" as used herein refers to an enzyme that decomposes hyaluronic acid. The hyaluronidase may be derived from sheep, cattle, mice, or humans. Specifically, the hyaluronidase may be human hyaluronidase (PH20) or recombinant human hyaluronidase (rHuPH20). The hyaluronidase may be, but is not limited to, a sequence of GenBank: NM_003117.4. Specifically, the hyaluronidase may have the amino acid sequence of SEQ ID NO: 135. In addition, specifically, the hyaluronidase gene may have the nucleotide sequence of SEQ ID NO: 136. In addition, the hyaluronidase gene may have a homology of about 90%, 91%, 92%, 93%, or 94% or more, preferably about 95%, 96%, 97%, 98%, or 99% or more, and most preferably about 99% or more, to the nucleotide sequence of SEQ ID NO: 136, as long as the function of the hyaluronidase is not modified.

In addition, the term "IL-12" as used herein is a molecule belonging to the cytokine family and is related to the immune system. The IL-12 may be the same as that obtained from any animal, such as humans (hIL-12), mice (mIL-12), horses, cattle, and pigs. The IL-12 may be, but is not limited to, a sequence disclosed in GenBank's NM_000882.3 (hIL-12A), AY008847.1 (hIL-12B), NM_001159424.2 (mIL-12α), or NM_001303244.1 (mIL-12β). Specifically, the IL-12 may be the amino acid sequence of SEQ ID NO: 137. Specifically, the IL-12 gene may include a linker represented by the nucleotide sequence of SEQ ID NO: 138, the nucleotide sequence of SEQ ID NO: 139 (p35 subunit), or the nucleotide sequence of SEQ ID NO: 140 (p40 subunit). In addition, the IL-12 gene may have a homology of about 90%, 91%, 92%, 93%, or 94% or more, preferably about 95%, 96%, 97%, 98%, or 99% or more, and most preferably about 99% or more, to the nucleotide sequence of SEQ ID NO: 138 to SEQ ID NO: 140, as long as the function of the IL-12 is not modified.

The recombinant vaccinia virus is a vaccinia virus comprising the sPD-1 gene, the hyaluronidase gene, and the IL-12 gene. Here, the sPD-1 gene, the hyaluronidase gene, and the IL-12 gene are inserted into the vaccinia virus' genome. In addition, the genes may also be inserted at different positions of the vaccinia virus' genome; however, such genes may be inserted at the same or similar positions. As an example, each of such genes may be inserted at the TK, K3L, or VGF position of the vaccinia virus. The recombinant vaccinia virus expresses sPD-1, hyaluronidase, and IL-12 in a host cell. The recombinant vaccinia virus is a vaccinia virus capable of expressing the sPD-1 gene, the hyaluronidase gene, and the IL-12 gene.

The aforementioned vaccinia virus may be a vaccinia virus having suppressed expression of any one selected from the group consisting of a gene encoding K3L, a gene encoding thymidine kinase (TK), a gene encoding vaccinia growth factor (VGF), and combinations thereof. Here, the combinations may refer to at least two of the gene encoding K3L, the gene encoding TK, and the gene encoding VGF.

For example, the recombinant vaccinia virus may be a vaccinia virus having the sPD-1 gene, the hyaluronidase gene, and the IL-12 gene, having suppressed expression of any one selected from the group consisting of the K3L gene, the TK gene, the VGF gene, and combinations thereof.

The term "VGF" as used herein refers to vaccinia growth factor. The vaccinia growth factor is an enzyme exhibiting a similar activity to epithelial growth factor. The vaccinia growth factor encoded by the VGF gene exhibits a growth factor activity in a case of being infected with the virus and may be synthesized at an initial stage of infection caused by the virus. The VGF may be a sequence of GenBank: AAO89288.1, ABD52455.1, or AIX98927.1, but is not limited thereto. Specifically, the VGF may have the amino acid sequence of SEQ ID NO: 141. In addition, the VGF gene may be the nucleotide sequence of SEQ ID NO: 142. The VGF may have a homology of about 70% or 75% or more, and preferably about 80%, 81%, 82%, 83%, 84%, 85%, 86%, 87%, 88%, or 89% or more, to the amino acid sequence of SEQ ID NO: 141. In addition, the VGF gene may have a homology of about 90%, 91%, 92%, 93%, or 94% or more, preferably about 95%, 96%, 97%, 98%, or 99% or more, and most preferably about 99% or more, to the nucleotide sequence of SEQ ID NO: 142.

The term "TK" as used herein refers to thymidine kinase. The thymidine kinase is an enzyme involved in biosynthesis of nucleotides. The thymidine kinase encoded by the TK gene causes a phosphoric acid at a γ position of ATP to bind to thymidine so that nucleotides constituting a viral DNA can be produced. The TK may be a sequence of GenBank: AAO89373.1, ABD52560.1, or AIX99011.1, but is not limited thereto. Specifically, the TK may have the amino acid sequence of SEQ ID NO: 143. The TK gene may be the nucleotide sequence of SEQ ID NO: 144. The TK may have a homology of about 70% or 75% or more, and preferably about 80%, 81%, 82%, 83%, 84%, 85%, 86%, 87%, 88%, or 89% or more, to the amino acid sequence of SEQ ID NO: 143. In addition, the TK gene may have a homology of about 90%, 91%, 92%, 93%, or 94% or more, preferably about 95%, 96%, 97%, 98%, or 99% or more, and most preferably about 99% or more, to the nucleotide sequence of SEQ ID NO: 144.

The term "K3L" as used herein refers to K3L protein. The K3L protein encoded by the K3L gene is a protein having a homology to translation initiation factor-2α (eIF-2α), and can suppress an action of protein kinase R (PKR) which is an interferon activator. The K3L may be a sequence of GenBank: AAO89313.1, ABD52483.1, or AGB75754.1, but is not limited thereto. Specifically, the K3L may have the amino acid sequence of SEQ ID NO: 145. The K3L gene may be the nucleotide sequence of SEQ ID NO: 146. The K3L may have a homology of about 70% or 75% or more, and preferably about 80%, 81%, 82%, 83%, 84%, 85%, 86%, 87%, 88%, or 89% or more, to the amino acid sequence of SEQ ID NO: 145. In addition, the K3L gene may have a homology of about 90%, 91%, 92%, 93%, or 94% or more, preferably about 95%, 96%, 97%, 98%, or 99% or more, and most preferably about 99% or more, to the nucleotide sequence of SEQ ID NO: 146.

Suppressed expression of a gene according to the present invention means that the gene is not expressed or only a part of the gene is expressed by partial or entire deletion of the gene or insertion of a foreign gene into the gene, so that an activity of a protein encoded by the gene is not exhibited. A method for deleting the gene or inserting a foreign gene may be performed by methods well known in the art. For example, this may be performed by methods for inserting a foreign gene which are disclosed in Molecular Cloning, A Laboratory Manual, Second Edition, by J. Sambrook, E. F. Fritsch and T. Maniatis (2003), Cold Spring Harbor Laboratory Press, Virology Methods Manual, edited by Brian W J Mahy and Hillar O Kangro (1996), Academic Press and Expression of genes by Vaccinia virus vectors, and Current Protocols in Molecular Biology, published by John Wiley and Son (1998), Chapter 16. Specifically, in an embodiment of the present invention, a foreign gene was inserted using pGEM-T Easy (Promega, Cat No. A1360) or pGEM-T (Promega, Cat No. A3600) plasmid system.

The vaccinia virus may be selected from, but is not limited to, the group consisting of Western Reserve (WR), New York Vaccinia Virus (NYVAC), Wyeth (The New York City Board of Health; NYCBOH), LC16m8, Lister, Copenhagen, Tian Tan, USSR, Tashkent, Evans, International Health Division-J (IHD-J), International Health Division-White (IHD-W), variants thereof, and combinations thereof. Specifically, the vaccinia virus may be WR, Lister, or IHD-W vaccinia virus, and may have a sequence of GenBank: AY243312.1, DQ121394.1, or AIX98951.1. In an embodiment of the present invention, the vaccinia virus may be IHD-W.

The term "V" or "virus V" as used herein refers to a recombinant vaccinia virus in which the vaccinia growth factor gene, VGF, is deleted, and the virus does not express the VGF gene due to deletion of the VGF gene.

In addition, the term "T" or "virus T" as used herein refers to a recombinant vaccinia virus in which thymidine kinase (TK) gene is deleted, and the virus does not express the TK gene due to deletion of the TK gene.

In addition, the term "K" or "virus K" as used herein refers to a recombinant vaccinia virus in which K3L gene is deleted, and the virus does not express the K3L gene due to deletion of the K3L gene.

In addition, the term "VT" or "virus VT" as used herein refers to a recombinant vaccinia virus in which the VGF and TK genes are deleted. Methods for inactivating expression of the vaccinia growth factor and the thymidine kinase are as described above.

In addition, the term "IHD-W-VV01" as used herein refers to a recombinant IHD-W vaccinia virus in which the VGF, TK, and K3L genes are deleted. Methods for inactivating expression of the vaccinia growth factor, thymidine kinase, and K3L proteins are as described above.

In addition, the term "IHD-W-VV02" as used herein refers to a recombinant IHD-W vaccinia virus in which the VGF, TK, and K3L genes are deleted and expression of the sPD1-Fc gene is induced.

In addition, the term "IHD-W-VV03" as used herein refers to a recombinant IHD-W vaccinia virus in which the VGF, TK, and K3L genes are deleted and expression of the PH20 gene is induced.

In addition, the term "IHD-W-VV04" as used herein refers to a recombinant IHD-W vaccinia virus in which the VGF, TK, and K3L genes are deleted and expression of the IL-12 gene is induced.

In addition, the term "IHD-W-VV05" as used herein refers to a recombinant IHD-W vaccinia virus in which the VGF, TK, and K3L genes are deleted and expression of the sPD 1-Fc and PH20 genes is induced.

In addition, the term "IHD-W-VV06" as used herein refers to a recombinant IHD-W vaccinia virus in which the VGF, TK, and K3L genes are deleted and expression of the sPD1-Fc, PH20, and IL-12 genes are induced.

Specifically, as an example of the recombinant vaccinia virus according to the present invention, the following may be mentioned. Variants of WR vaccinia virus may include WR vaccinia viruses in which expression of at least one of the VGF, TK, and K3L genes is suppressed, and expression of the sPD1-Fc, PH20, and IL-12 genes is induced.

In addition, variants of NYVAC vaccinia virus may include NYVAC vaccinia viruses in which expression of at least one of the VGF, TK, and K3L genes is suppressed, and expression of the sPD 1-Fc, PH20, and IL-12 genes is induced.

In addition, variants of Wyeth vaccinia virus may include Wyeth vaccinia viruses in which expression of at least one of the VGF, TK, and K3L genes is suppressed, and expression of the sPD 1-Fc, PH20, and IL-12 genes is induced.

In addition, variants of Lister vaccinia virus may include Lister vaccinia viruses in which expression of at least one of the VGF, TK, and K3L genes is suppressed, and expression of the sPD 1-Fc, PH20, and IL-12 genes is induced.

In addition, variants of Tian tan vaccinia virus may include Tian tan vaccinia viruses in which expression of at least one of the VGF, TK, and K3L genes is suppressed, and expression of the sPD 1-Fc, PH20, and IL-12 genes is induced.

In addition, variants of USSR vaccinia virus may include USSR vaccinia viruses in which expression of at least one of the VGF, TK, and K3L genes is suppressed, and expression of the sPD 1-Fc, PH20, and IL-12 genes is induced.

In addition, variants of Tashkent vaccinia virus may include Tashkent vaccinia viruses in which expression of at least one of the VGF, TK, and K3L genes is suppressed, and expression of the sPD 1-Fc, PH20, and IL-12 genes is induced.

In addition, variants of IHD-J vaccinia virus may include IHD-J vaccinia viruses in which expression of at least one of the VGF, TK, and K3L genes is suppressed, and expression of the sPD 1-Fc, PH20, and IL-12 genes is induced.

Furthermore, variants of IHD-W vaccinia virus may include IHD-W vaccinia viruses in which expression of at least one of the VGF, TK, and K3L genes is suppressed, and expression of the sPD 1-Fc, PH20, and IL-12 genes is induced.

In another aspect of the present invention, there is provided a pharmaceutical composition for use in preventing or treating cancer, comprising, as an active ingredient, the recombinant vaccinia virus according to the present invention. Here, as described above, the recombinant vaccinia virus may be a vaccinia virus in which expression of the sPD1-Fc, PH20, and IL-12 genes, is induced, and expression of the VGF, TK, or K3L gene, or combinations thereof is suppressed. The sPD1-Fc, PH20, IL-12, VGF, TK, and K3L genes are as described above.

The term "cancer" as used herein may be solid cancer or blood cancer. Here, the solid tumor may be selected from the group consisting of lung cancer, colorectal cancer, prostate cancer, thyroid cancer, breast cancer, brain cancer, head and neck cancer, fibrosarcoma, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, and combinations thereof. According to an embodiment of the present invention, the cancer may be lung cancer, liver cancer, prostate cancer, head and neck cancer, fibrosarcoma, brain cancer, breast cancer, ovarian cancer, pancreatic cancer, skin cancer, or colorectal cancer. In addition, the blood cancer may be selected from the group consisting of lymphoma, acute leukemia, multiple myeloma, and combinations thereof.

The pharmaceutical composition of the present invention may further contain one or more pharmaceutically acceptable additives selected from the group consisting of excipients, lubricants, wetting agents, sweeteners, fragrances, and preservatives.

The composition of the present invention may be formulated according to a conventional method. The composition of the present invention may be formulated employing a method known in the art so as to provide rapid, sustained, or delayed release of an active ingredient, in particular after being administered to a mammal. Depending on the formulation, the composition of the present invention may be appropriately administered to an individual. Such administration may be parenteral administration, and examples thereof may include intratumoral, intradermal, intramuscular, intraperitoneal, intravenous, intraarterial, subcutaneous, intranasal, epidural, and oral route. A form of a preparation for parenteral administration may be an injectable preparation.

The individual may be a mammal, and the mammal may include, but is not limited to, primates (for example, humans), cattle, sheep, goats, horses, pigs, dogs, cats, rabbits, rats, mice, fish, birds, and the like. Specifically, the mammal may be a human. The composition of the present invention may be appropriately administered by a person skilled in the art depending on patient's age, sex, weight, severity of disease symptom, and route of administration. The administration may be once a day or several times a day, and may be repeatedly administered on an appropriate cycle.

A preferred dosage of a composition that contains the recombinant vaccinia virus of the present invention varies depending on condition and weight of an individual, severity of disease, drug form, route and duration of administration, and may be appropriately selected by a person skilled in the art. Specifically, the dosage may be such that a patient receives virus particles, virus units having infectivity (TCID₅₀), or plaque forming units (pfu) of 1×10⁵ to 1×10¹⁸, and preferably 1×10⁵, 2×10⁵, 5×10⁵, 1×10⁶, 2×10⁶, 5×10⁶, 1×10⁷, 2×10⁷, 5×10⁷, 1×10⁸ 2×10⁸, 5×10⁸, 1×10⁹, 2×10⁹, 5×10⁹, 1×10¹⁰, 5×10¹⁰, 1×10¹¹, 5×10¹¹, 1×10¹², 1×10¹³, 1×10¹⁴, 1×10¹⁵, 1×10¹⁶, 1×10¹⁷ or more, in which various values and ranges therebetween may be included. In addition, a dosage of virus may be 0.1 ml, 1 ml, 2 ml, 3 ml, 4 ml, 5 ml, 6 ml, 7 ml, 8 ml, 9 ml, 10 ml or more, and all values and ranges therebetween may be included.

### Mode for the Invention

Hereinafter, the present invention will be described in more detail with reference to the following examples. However, the following examples are intended to merely illustrate the present invention, and the present invention is not limited thereto.

### I. Production of recombinant vaccinia virus

The present inventors constructed recombinant vaccinia viral plasmids in which TK, VGF, and K3L genes are deleted or expression thereof is inactivated. Using these plasmids, recombinant vaccinia viruses in which expression of the above genes is suppressed were produced and comparison was made for properties thereof as anti-cancer substances. In addition, the present inventors constructed recombinant vaccinia viral plasmids obtained by insertion of sPD1-Fc, PH20, IL-12 genes into the above recombinant vaccinia viral plasmids. Using these plasmids, recombinant vaccinia viruses in which expression of the above genes is induced were produced and comparison was made for properties thereof as anti-cancer substances.

### Example 1. Production of recombinant IHD-W vaccinia virus

### Example 1.1. Production of IHD-W-VV01 virus

### Example 1.1.1. Construction of recombinant IHD-W vaccinia viral plasmid in which VGF gene is deleted

First, genetic regions that flank VGF gene on both sides in genomic DNA of IHD-W vaccinia virus (ATCC, Cat No. VR-1441) were amplified by PCR. Here, information on primers used for the amplification of homologous nucleotide sequences that flank the VGF gene on both sides is shown in Table 4 below. In such a manner, VGF-L(IHD-W) and VGF-R(IHD-W) fragments were obtained, and then ligated with a pGEM-T Easy plasmid to construct pGEM-T Easy-VGF-L(IHD-W) or pGEM-T Easy-VGF-R(IHD-W).

**[Table 4]**

| Name | Sequence (5'→3') | SEQ ID NO |
|---|---|---|
| VGF-L forward (IHD-W) | CGAAAGCTTGTAAGATGTTTAGAAAATGGATATTTC | SEQ ID NO: 1 |
| VGF-L reverse (IHD-W) | CTGGGATCCTAGGCTAGCGTGTAAATAATATAAAAATAAC AATACAATATTG | SEQ ID NO: 2 |
| VGF-R forward (IHD-W) | CTGGAATTCGATTTAATTAATTTTTATAAATTTTTTTTATGA GTATTTTTACAAAAAA | SEQ ID NO: 3 |
| VGF-R reverse (IHD-W) | TAGGGATCCCATCGATAGTACAATAACTTTTATGAAAT | SEQ ID NO: 4 |

The pGEM-T Easy-VGF-R(IHD-W) and pSP72 were respectively treated with EcoRI and BglII, and then ligated to construct pSP72-VGF-R(IHD-W). The constructed pSP72-VGF-R(IHD-W) and the pGEM-T Easy-VGF-L(IHD-W) were respectively treated with HindIII and BamHI, and ligated to construct pSP72-VGF-L-VGF-R(IHD-W).

Next, in order to introduce p11 promoter and LacZ gene into the pSP72-VGF-L-VGF-R(IHD-W), a p11-LacZ expression cassette in the WR VGF(i) shuttle plasmid as constructed in Example 2.1.1 below, and the pSP72-VGF-L-VGF-R(IHD-W) were treated with NheI and PacI, and ligated to construct pSP72-VGF-L-p11-LacZ-VGF-R(IHD-W) (hereinafter referred to as "IHD-W VGF shuttle plasmid").

### Example 1.1.2. Construction of recombinant IHD-W vaccinia viral plasmid in which TK gene is deleted

First, in order to obtain genetic regions that flank TK gene on both sides in genomic DNA of IHD-W vaccinia virus, PCR was performed, and as a result, TK-L(IHD-W) and TK-R(IHD-W) fragments were obtained. Here, primers used are shown in Table 5 below. The obtained TK-R(IHD-W) fragment and pSP72 plasmid were respectively treated with EcoRI and BglII, and ligated to construct pSP72-TK-R(IHD-W). In addition, the pSP72-TK-R(IHD-W) and the TK-L(IHD-W) fragment were respectively treated with PstI and BamHI, and ligated to construct pSP72-TK-L-TK-R(IHD-W).

The constructed pSP72-TK-L-TK-R(IHD-W) plasmid and pSP72-TK-L-EGFP-pSE/L-p7.5-TK-R(WR), which is the WR TK(i) shuttle plasmid as constructed in Example 2.1.2 below, were respectively treated with EcoRI and NotI, and ligated to finally construct pSP72-TK-L-TF-EGFP-pSE/L-p7.5-Gpt-TK-R(IHD-W) (hereinafter referred to as "IHD-W TK shuttle plasmid").

**[Table 5]**

| Name | Sequence (5'→3') | SEQ ID NO |
|---|---|---|
| TK-L forward (IHD-W) | GATCTGCAGCCCTCTTCAAGAACCCATTAG | SEQ ID NO: 5 |
| TK-L reverse (IHD-W) | TAGGGATCCTAGGCGGCCGCATGACAATAAAGAATTAATT ATTGTTCACTT | SEQ ID NO: 6 |
| TK-R forward (IHD-W) | CATGAATTCTATTATATTTTTTATCTAAAAAACTAAAAATAA ACAT | SEQ ID NO: 7 |
| TK-R reverse (IHD-W) | AGATCTATCGCTTTAGTAGTAGGAAATGTTTTATTG | SEQ ID NO: 8 |

### Example 1.1.3. Construction of recombinant IHD-W vaccinia viral plasmid in which K3L gene is deleted

First, genetic regions that flank K3L gene on both sides in genomic DNA of IHD-W vaccinia virus were amplified by PCR, and then respectively inserted into pGEM-T Easy, to construct pGEM-T Easy-K3L-L(IHD-W) and pGEM-T Easy-K3L-R(IHD-W). Here, primers used are shown in Table 6 below.

In order to construct a gene expression cassette inside the K3L shuttle plasmid, p7.5-DsRed gene cassette was amplified by PCR using, as a template, the WR K3L(i) shuttle plasmid as constructed in Example 2.1.3 below, and then inserted into pGEM-T Easy to construct pGEM-T Easy-p7.5-DsRed. Sequences of primers used for the amplification of the p7.5 promoter and the DsRed gene are shown in Table 6 below.

**[Table 6]**

| Name | Sequence (5'→3') | SEQ ID NO |
|---|---|---|
| K3L-L forward (IHD-W) | TCGGTCGACCATATGTTTAAACGACGCATTATCTG | SEQ ID NO: 9 |
| K3L-L reverse (IHD-W) | TCGAAGCTTTTTTTATACCGAACATAAAAATAAGGTTAATTAT | SEQ ID NO: 10 |
| K3L-R forward (IHD-W) | CGCAGATAAAAATCACTTGTTAACGGGCTCGTAA | SEQ ID NO: 11 |
| K3L-R reverse (IHD-W) | AAGCGCTAACATGGATTAGGAAGCGCTAACATGG | SEQ ID NO: 12 |
| p7.5-DsRed forward | AGGAAGCTTTCCAAACCCACCCGCTTTTTAT | SEQ ID NO: 13 |
| p7.5-DsRed reverse | CGGATATCTTTTTATCTGCGCGGTTAAC | SEQ ID NO: 14 |

The constructed pGEM-T Easy-p7.5-DsRed and pSP72 plasmid were respectively treated with HindIII and EcoRV, and then ligated to complete pSP72-p7.5-DsRed. In addition, the constructed pSP72-p7.5-DsRed and the above-constructed pGEM-T Easy-K3L-R(IHD-W) were treated with EcoRV and BamHI, and then ligated to construct pSP72-p7.5-DsRed-K3L-R(IHD-W). Next, the constructed pSP72-p7.5-DsRed-K3L-R(IHD-W) and the pGEM-T Easy-K3L-L(IHD-W) were respectively treated with SalI and HindIII, and then ligated to finally construct pSP72-K3L-L-p7.5-DsRed-K3L-R(IHD-W) (hereinafter referred to as "IHD-W K3L shuttle plasmid").

### Example 1.1.4. Production of recombinant IHD-W vaccinia virus in which VGF gene is deleted

Using the IHD-W VGF shuttle plasmid as constructed in Example 1.1.1., a recombinant IHD-W vaccinia virus in which VGF gene is deleted was produced by the following method.

In order to obtain a recombinant virus, HeLa cells were prepared in a 6-well plate at a condition of 3×10⁵ cells/well and in a state of MEM medium containing 2% fetal bovine serum. Then, the HeLa cells were transfected with 2 µg of the IHD-W K3L shuttle plasmid using jetPRIME and simultaneously treated with IHD-W wild-type vaccinia virus at 0.05 MOI. After 4 hours of incubation, the medium was replaced with MEM medium containing 5% fetal bovine serum, and then the cells were further incubated for 48 hours. Finally, the infected cells were collected with 500 µl of the medium, and then the cells were lysed by repeating freezing and thawing three times, to obtain crude viruses. The crude viruses were used and repeatedly subjected to plaque isolation by a conventional method, so that purely isolated recombinant IHD-W vaccinia virus V was obtained.

### Example 1.1.5. Production of recombinant IHD-W vaccinia virus in which VGF and TK genes are deleted

Recombinant IHD-W vaccinia virus VT in which VGF and TK genes are deleted was obtained in the same conditions and methods as in Example 1.1.4., except that the IHD-W TK shuttle plasmid as constructed in Example 1.1.2. and the recombinant IHD-W vaccinia virus V as produced in Example 1.1.4. were used.

### Example 1.1.6. Production of IHD-W-VV01 virus

Recombinant IHD-W-VV01 in which VGF, TK, and K3L genes are deleted was obtained in the same conditions and methods as in Example 1.1.4., except that the IHD-W K3L shuttle plasmid as constructed in Example 1.1.3. and the recombinant IHD-W vaccinia virus VT as produced in Example 1.1.5. were used; and the gene structure thereof is illustrated in Fig. 1.

### Example 1.2. Production of IHD-W-VV02 virus

### Example 1.2.1. Construction of recombinant IHD-W vaccinia viral plasmid in which TK gene is deleted and expression of sPD1-Fc gene is induced

In order to construct a gene expression cassette inside the TK shuttle plasmid, sPD1-Fc gene was amplified by PCR using, as a template, the pE3.1(EF1a.sPD-1.Fc) plasmid (provided by the department of urologic cancer research at the National Cancer Center), and then inserted into pGEM-T easy to construct pGEM-T easy-sPD1-Fc. In addition, p7.5 promoter was amplified by PCR using, as a template, pGL4.1 p7.5 luciferase plasmid, and then inserted into pGEM-T easy, to construct pGEM-T easy-p7.5. The constructed pGEM-T easy-p7.5 and pGEM-T easy-sPD1-Fc plasmids were respectively treated with PacI and NheI, and then ligated to complete pGEM-T easy-p7.5-sPD1-Fc. Sequences of primers used for the amplification of the p7.5 promoter and the sPD1-Fc gene are shown in Table 7 below. The IHD-W TK shuttle plasmid as constructed in Example 1.1.2. was treated with EcoRI and SalI, and then the p7.5-sPD1-Fc gene cassette was inserted thereinto by an infusion cloning method, to finally construct pSP72-TK-L-TF-pSE/L-EGFP-TF-TF-sPD1-Fc-p7.5-TK-R(IHD-W) (hereinafter referred to as "IHD-W TK(sPD1-Fc) shuttle plasmid"). Sequences of primers used for the infusion cloning process are shown in Table 7 below.

**[Table 7]**

| Name | Sequence (5'→3') | SEQ ID NO |
|---|---|---|
| sPD 1-Fc forward | AGTGAATTCAGTGCTAGCAGTAAGCTTATGTGGGTCCGGC AGGTAC | SEQ ID NO: 15 |
| sPD1-Fc reverse | ACTTTAATTAATCATTTACCCGGAGTCCGGG | SEQ ID NO: 16 |
| p7.5 forward | GAATTCATCGAGCTCTCCAAACCCACCCGCTTTTTA | SEQ ID NO: 17 |
| p7.5 reverse | ATTAATTAAATACTAGTGATGCTAGCGATGGATCCCCGTGC AATAAATTAGAATATATTTTC | SEQ ID NO: 18 |
| Infusion forward (sPD1-Fc) | AAATATAATAGAATTCGATTTGCTAGCTCCAAAC | SEQ ID NO: 19 |
| Infusion reverse (sPD1-Fc) | TGGGCCCTATGTCGACGGATCCCGAGAAA | SEQ ID NO: 20 |

### Example 1.2.2. Production of recombinant IHD-W vaccinia virus in which VGF and K3L genes are deleted

Recombinant IHD-W vaccinia virus VK in which VGF and K3L genes are deleted was obtained in the same conditions and methods as in Example 1.1.4., except that the IHD-W K3L shuttle plasmid as constructed in Example 1.1.3. and the recombinant IHD-W vaccinia virus V as produced in Example 1.1.4. were used.

### Example 1.2.3. Production of IHD-W-VV02 virus

Recombinant IHD-W-VV02, in which VGF, TK, and K3L genes are deleted and expression of sPD1-Fc gene is induced, was obtained in the same conditions and methods as in Example 1.1.4., except that the IHD-W TK(sPD1-Fc) shuttle plasmid as constructed in Example 1.2.1. and the recombinant IHD-W vaccinia virus VK as produced in Example 1.2.2. were used; and the gene structure thereof is illustrated in Fig. 2.

### Example 1.2.4. Identification of insertion and expression of sPD1-Fc gene in IHD-W-VV02

gDNA of recombinant IHD-W-VV02 as produced in Example 1.2.3. was obtained using the Nucleospin prep kit (Cat # 740956250), and the structure of gDNA of the recombinant virus was identified using the primers in Table 8 below. As illustrated in Fig. 3, the results obtained by identifying the DNA structure at each of the VGF, TK, and K3L positions showed that there are changes in PCR fragment sizes at the positions where the genes have been inserted, as compared with a control group.

Western blotting was performed to identify that the inserted sPD1-Fc gene is expressed, via the recombinant virus, in infected cells. First, HeLa cells were treated with recombinant IHD-W-VV02 at 0.05 MOI for 44 to 52 hours. Then, the medium was replaced with MEM medium containing no fetal bovine serum, and the cells were further incubated for 16 to 20 hours. The medium and the cells were separated from each other, and proteins were obtained therefrom, respectively. The prepared proteins were quantified by the Bradford assay method, and then Western blotting was used to identify the protein expression. Here, anti-mPdcd1 (E-18) (Santacruz, Cat # SC-10299) was used as a primary antibody, and anti-Goat was used as a secondary antibody. The expression results are illustrated in Fig. 4.

**[Table 8]**

| Name | Sequence (5'→3') | SEQ ID NO |
|---|---|---|
| VGF forward | AGGTTCCGTAAGCAAAGAATATAAG | SEQ ID NO: 21 |
| VGF reverse | AGGTAACTAGATTTTACTTTAATATGCCGAA | SEQ ID NO: 22 |
| TK forward | GGAAGGGTCAAACTTAATAAAGGAT | SEQ ID NO: 23 |
| TK reverse | ACCGTGTCGCTGTAACTTACTA | SEQ ID NO: 24 |
| K3L forward | GACGCTGAACACGTTAACGATAGT | SEQ ID NO: 25 |
| K3L reverse | ACTGCGACGAGATACAACCGGA | SEQ ID NO: 26 |

### Example 1.3. Production of IHD-W-VV03 virus

### Example 1.3.1. Construction of recombinant IHD-W vaccinia viral plasmid in which TK gene is deleted and expression of PH20 gene is induced

pHyb promoter DNA was synthesized from pGL4.1-pHyb plasmid using the primers shown in Table 9 below, and then inserted into pGEM-T easy vector by an infusion cloning method, to construct pGEM-T easy-pHyb. In order to insert PH20 gene into the constructed pGEM-T Easy-pHyb, PH20 Exon2, Exon3, and Exon4 were respectively synthesized from gDNA of A549 cells through a PCR process. Here, the primers used are shown in Table 9 below.

First, in order to insert PH20-Exon2, the pGEM-T easy-pHyb plasmid was treated with Spe1. The PH20-Exon2 was synthesized through a PCR process, and then inserted into linearized pGEM-T easy-pHyb by an infusion cloning method, to construct pGEM-T easy-pHyb-PH20-Exon2 plasmid. The constructed pGEM-T Easy-pHyb-PH20-Exon2 plasmid was treated with SacI; and PH20-Exon3 and PH20-Exon4 were synthesized through a PCR process, and then inserted thereinto by an infusion cloning method, to construct pGEM-T easy-pHyb-PH20. The IHD-W TK shuttle plasmid as constructed in Example 1.1.2. was treated with EcoRI and SalI, and then the pHyb-PH20 gene cassette was inserted thereinto by an infusion cloning method, to finally construct pSP72-TK-L-TF-pSE/L-EGFP-TF-pHyb-PH20-TF-TK-R(IHD-W) (hereinafter referred to as "IHD-W TK(PH20) shuttle plasmid"). Sequences of primers used in the infusion cloning process are shown in Table 9 below.

**[Table 9]**

| Name | Sequence (5'→3') | SEQ ID NO |
|---|---|---|
| pHyb promoter forward | TGTCGACCTAGCACGCGTGTTTAAACGTT | SEQ ID NO: 27 |
| pHyb promoter reverse | CAACAGTACCGGATTGCCAA | SEQ ID NO: 28 |
| Exon2 forward | ACTGTTGAATACTAGATGGGAGTGCTAAAATTCAAGCAC | SEQ ID NO: 29 |
| Exon2 reverse | CCGCGAATTCACTAGAGCTCTTGAGAAAGGAATTTCAAAAC TTGAT | SEQ ID NO: 30 |
| Exon3 forward | AATTCCTTTCTCAAGATGAACTTGTGTATACATTTGGC | SEQ ID NO: 31 |
| Exon3 reverse | GAGCAAGCAAGATTTCATACTTCGCATTATACTGAGGGTT | SEQ ID NO: 32 |
| Exon4 forward | AAATCTTGCTTGCTCCTAGACAATT | SEQ ID NO: 33 |
| Exon4 reverse | ATCCAACGCGTTGGGATCCGCATAAAAATGCATAAAAATTTA TAGTGTGGAGGGTGAAGCATTG | SEQ ID NO: 34 |
| Infusion forward (PH20) | TGGGCCCTATGTCGACCTAGCACGCGTGTTTAAA | SEQ ID NO: 35 |
| Infusion reverse (PH20) | AAATATAATAGAATTCGCTAGCGTTGGGATCCGCATAA | SEQ ID NO: 36 |

### Example 1.3.2. Production of IHD-W-VV03 virus

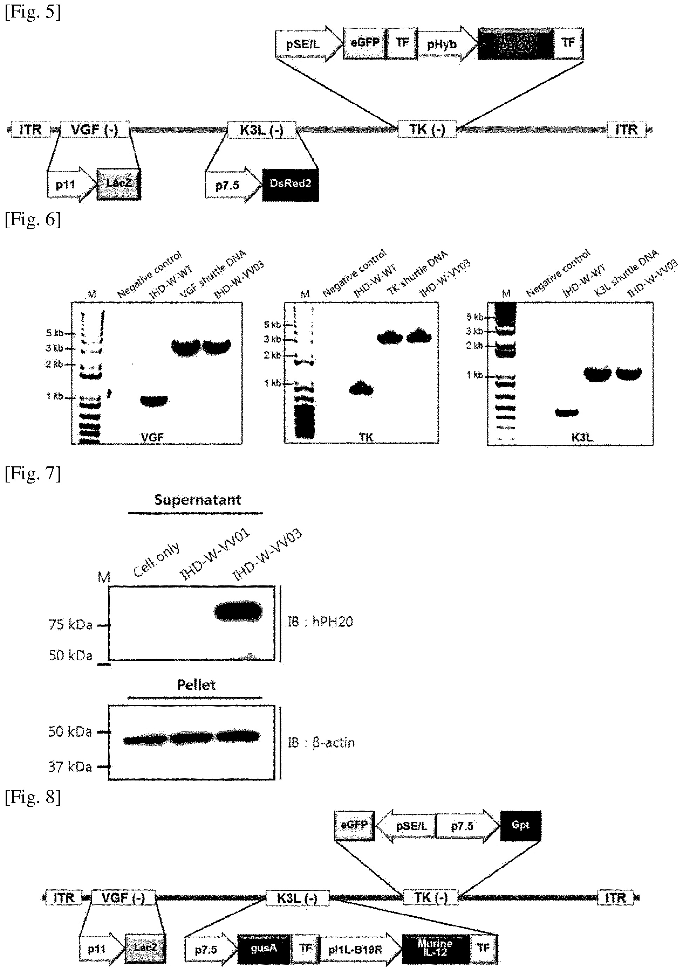

Recombinant IHD-W-VV03, in which VGF, TK, and K3L genes are deleted and expression of PH20 gene is induced, was obtained in the same conditions and methods as in Example 1.1.4., except that the IHD-W TK(PH20) shuttle plasmid as constructed in Example 1.3.1. and the recombinant IHD-W vaccinia virus VK as produced in Example 1.2.2. were used; and the gene structure thereof is illustrated in Fig. 5.

### Example 1.3.3. Identification of insertion and expression of PH20 gene in IHD-W-VV03

gDNA of recombinant IHD-W-VV03 as produced in Example 1.3.2. was obtained using the Nucleospin prep kit (Cat # 740956250), and the structure of gDNA of the recombinant virus was identified using the primers in Table 10 below. As illustrated in Fig. 6, the results obtained by identifying the DNA structure at each of the VGF, TK, and K3L positions showed that there are changes in PCR fragment sizes at the positions where the genes have been inserted, as compared with a control group.

Western blotting was performed to identify that the inserted PH20 gene is expressed, via the recombinant virus, in infected cells. First, HeLa cells were treated with recombinant IHD-W-VV03 at 0.05 MOI for 44 to 52 hours. Then, the medium was replaced with MEM medium containing no fetal bovine serum, and the cells were incubated for 16 to 20 hours. The medium and the cells were separated from each other, and proteins were obtained therefrom, respectively. The prepared proteins were quantified by the Bradford assay method, and then Western blotting was used to identify the protein expression. Here, anti-hPH20 (LsBio, Cat # LS-C331909) was used as a primary antibody, and anti-Rabbit was used as a secondary antibody. The expression results are illustrated in Fig. 7.

**[Table 10]**

| Name | Sequence (5'→3') | SEQ ID NO |
|---|---|---|
| VGF forward | AGGTTCCGTAAGCAAAGAATATAAG | SEQ ID NO: 37 |
| VGF reverse | AGGTAACTAGATTTTACTTTAATATGCCGAA | SEQ ID NO: 38 |
| TK forward | GGAAGGGTCAAACTTAATAAAGGAT | SEQ ID NO: 39 |
| TK reverse | ACCGTGTCGCTGTAACTTACTA | SEQ ID NO: 40 |
| K3L forward | GACGCTGAACACGTTAACGATAGT | SEQ ID NO: 41 |
| K3L reverse | ACTGCGACGAGATACAACCGGA | SEQ ID NO: 42 |

### Example 1.4. Production of IHD-W-VV04 virus

### Example 1.4.1. Construction of recombinant IHD-W vaccinia viral plasmid in which K3L gene is deleted and expression of IL-12 gene is induced

pGL4.1-pI1L-B19R plasmid as a DNA template was amplified with the primers in Table 11 below to synthesize pI1L-B19R promoter DNA, and then insertion thereof was performed by an infusion cloning method to construct pGEM-T Easy-pI1L-B19R plasmid. In order to insert, into the plasmid, IL-12 gene that encodes a single peptide, IL-12 p40 and p35 subunit genes were amplified with the primers in Table 11 below using pVAX1-IL-12 plasmid as a DNA template. Here, a sequence constituting a linker was inserted into the 5' end of reverse primer p40 and the 3' end of forward primer of p35. The IL-12 gene amplified in the above was inserted into the pGEM-T Easy-pI1L-B19R plasmid, which had been linearized with XhoI, by an infusion cloning method, to construct pGEM-T Easy-pI1L-B19R-IL-12 plasmid. The above-constructed pGEM-T Easy-pI1L-B19R-IL-12 plasmid and the IHD-W K3L shuttle plasmid as constructed in Example 1.1.3. were respectively treated with NotI, and then ligated to construct pSP72-K3L-L-p7.5-DsRed-pI1L-B19R-IL-12-TF-TF-K3L-R plasmid.

PCR was performed from DH1 gDNA with the primers in Table 11 below, and the obtained gusA marker gene was inserted into pGEM-T easy to construct pGEM-T easy-gusA. GusA fragments were obtained with the primers in Table 11 below using the plasmid as a template. The above-constructed pSP72-K3L-L-p7.5-DsRed-pI1L-BI9R-IL-12-TF-TF-K3L-R plasmid was treated with BamHI and SacII, and then infusion cloning was performed to finally construct pSP72-K3L-L-p7.5-gusA-TF-pI1L-B19R-IL-12-TF-TF-K3L-R (hereinafter referred to as "IHD-W K3L(IL-12) shuttle plasmid"). Sequences of primers used in the above process are shown in Table 11 below.

**[Table 11]**

| Name | Sequence (5'→3') | SEQ ID NO |
|---|---|---|
| pI1L-B19R promoter forward | ACTGTCGACTTTGTATTTAAAAGTTGTTTGGTGAACTT | SEQ ID NO: 43 |
| pI1L-B19R promoter reverse | GTATCTCGAGTAGGCCTTTATACGGCACTAAAATATTTA TATAATATC | SEQ ID NO: 44 |
| Infusion forward (IL-12 P40) | TAAAGGCCTACTCGACATGTGTCCTCAGAAGCTAACC ATC | SEQ ID NO: 45 |
| Infusion reverse (IL-12 P40) | CGACCCACCACCGCCCGAGCCACCGCCACCGGATCGG ACCCTGCAGGGA | SEQ ID NO: 46 |
| Infusion forward (IL-12 P35) | GGCGGTGGTGGGTCGGGTGGCGGCGGATCTAGGGTCA TTCCAGTCTCTGGAC | SEQ ID NO: 47 |
| Infusion reverse (IL-12 P35) | GTGATTGTATCTCGAGTAGTCGACCTATAAAAACCTAT AAAAACTCAGGCGGAGCTCAGATAGC | SEQ ID NO: 48 |
| PCR forward (gusA) | ATCTGCAGAAGCTTCAATACTCGAGATGTTACGTCCTG TAGAAACCC | SEQ ID NO: 49 |
| PCR reverse (gusA) | CGTTCTAGAGTTAATTAATCATTGTTTGCCTCCCTGCTG | SEQ ID NO: 50 |
| Infusion forward (gusA) | TATTGCACGGGGATCAGCTCGAGATGTTACGTCCT | SEQ ID NO: 51 |
| Infusion reverse (gusA) | AGTAATCGAATTCCCagaaaaatTCATTGTTTGCCTCC | SEQ ID NO: 52 |

### Example 1.4.2. Production of IHD-W-VV04 virus

Recombinant IHD-W-VV04, in which VGF, TK, and K3L genes are deleted and expression of IL-12 gene is induced, was obtained in the same conditions and methods as in Example 1.1.4., except that the IHD-W K3L(IL-12) shuttle plasmid as constructed in Example 1.4.1. and the recombinant IHD-W vaccinia virus VT as produced in Example 1.1.5. were used; and the gene structure thereof is illustrated in Fig. 8.

### Example 1.4.3. Identification of insertion and expression of IL-12 gene in IHD-W-VV04

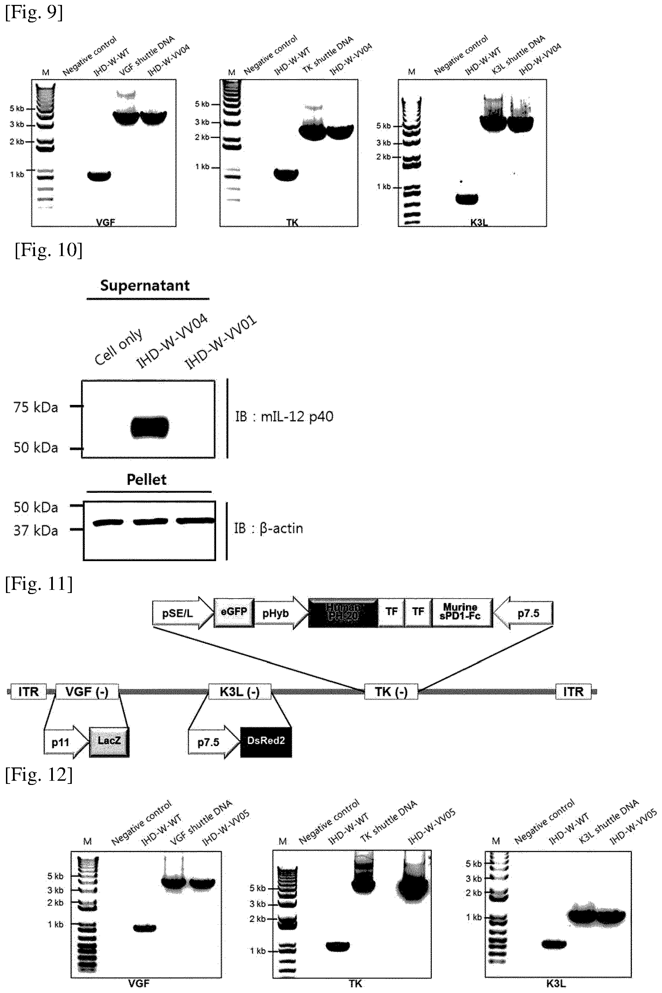

gDNA of recombinant IHD-W-VV04 as produced in Example 1.4.2. was obtained using the Maxwell purification kit (Cat # AS 1330), and the structure of gDNA of the recombinant virus was identified using the primers in Table 12 below. As illustrated in Fig. 9, the results obtained by identifying the DNA structure at each of the VGF, TK, and K3L positions showed that there are changes in PCR fragment sizes at the positions where the genes have been inserted, as compared with a control group.

Western blotting was performed to identify that the inserted IL-12 gene is expressed, via the recombinant virus, in infected cells. First, HeLa cells were treated with recombinant IHD-W-VV04 at 0.05 MOI for 44 to 52 hours. Then, the medium was replaced with MEM medium containing no fetal bovine serum, and the cells were incubated for 16 to 20 hours. The medium and the cells were separated from each other, and proteins were obtained therefrom, respectively. The prepared proteins were quantified by the Bradford assay method, and then Western blotting was used to identify the protein expression. Here, anti-mIL-12/IL-23 p40 (R&D Systems, Cat # MAB4991) was used a primary antibody, and anti-Rat was used as a secondary antibody. The expression results are illustrated in Fig. 10.

**[Table 12]**

| Name | Sequence (5'→3') | SEQ ID NO |
|---|---|---|
| VGF forward | AGGTTCCGTAAGCAAAGAATATAAG | SEQ ID NO: 53 |
| VGF reverse | AGGTAACTAGATTTTACTTTAATATGCCGAA | SEQ ID NO: 54 |
| TK forward | GGAAGGGTCAAACTTAATAAAGGAT | SEQ ID NO: 55 |
| TK reverse | ACCGTGTCGCTGTAACTTACTA | SEQ ID NO: 56 |
| K3L forward | CGAATAGCCAAACGGCGAGAAG | SEQ ID NO: 57 |
| K3L reverse | ACGGATACATGTGGAGCATCTATTA | SEQ ID NO: 58 |

### Example 1.5. Production of IHD-W-VV05 virus

### Example 1.5.1. Construction of recombinant IHD-W vaccinia viral plasmid in which TK gene is deleted and expression of sPD1-Fc and PH20 genes is induced

The IHD-W TK shuttle plasmid as constructed in Example 1.1.2. and the pGEM-T Easy-p7.5-sPD1-Fc plasmid were treated with NheI and SalI, and then ligated to construct pSP72-TK-L-TF-TF-sPD1-Fc-p7.5-TK-R(IHD-W) plasmid. The plasmid and the pGEM-T Easy-pHyb-PH20 plasmid were treated with SalI and BamHI, and ligated to construct pSP72-TK-L-TF-pHyb-PH20-TF-TF-sPD1-Fc-p7.5-TK-R(IHD-W). In order to insert the marker gene EGFP into the plasmid, pSE/L-EGFP gene was synthesized through a PCR process using the IHD-W TK shuttle plasmid as a template. Here, the primers used are shown in Table 13 below. The pSP72 TK-L-TF-pHyb-PH20-TF-TF-sPD1-Fc-p7.5-TK-R(IHD-W) shuttle plasmid was treated with PacI, and the synthesized pSE/L-EGFP gene was inserted thereinto by an infusion cloning method, to construct pSP72-TK-L-TF-pSE/L-EGFP-TF-pHyb-PH20-TF-TF-sPD1-Fc-p7.5-TK-R(IHD-W) (hereinafter referred to as "IHD-W TK(sPD1-Fc + PH20) shuttle plasmid").

**[Table 13]**

| Name | Sequence (5'→3') | SEQ ID NO |
|---|---|---|
| pSE/L-EGFP forward | CAGATAAAAATTAATTAAAAAAATTGAAATTTTATTTTTTTT TTTTGGAATATAA | SEQ ID NO: 59 |
| pSE/L-EGFP reverse | AGAGCTCAGATTAATATCGATTTACTTGTACAGCTCGTCCAT G | SEQ ID NO: 60 |

### Example 1.5.2. Production of IHD-W-VV05 virus

Recombinant IHD-W-VV05, in which VGF, TK, and K3L genes are deleted and expression of sPD1-Fc and PH20 genes is induced, was obtained in the same conditions and methods as in Example 1.1.4., except that the IHD-W TK(sPD1-Fc + PH20) shuttle plasmid as constructed in Example 1.5.1. and the recombinant IHD-W vaccinia virus VK as produced in Example 1.2.2. were used; and the gene structure thereof is illustrated in Fig. 11.

### Example 1.5.3. Identification of insertion and expression of sPD1-Fc and PH20 genes in recombinant IHD-W vaccinia virus IHD-W-VV05

gDNA of recombinant IHD-W-VV05 as produced in Example 1.5.2. was obtained using the Nucleospin prep kit (Cat # 740956250), and the structure of gDNA of the recombinant virus was identified using the primers in Table 14 below. As illustrated in Fig. 12, the results obtained by identifying the DNA structure at each of the VGF, TK, and K3L positions showed that there are changes in PCR fragment sizes at the positions where the genes have been inserted, as compared with a control group.

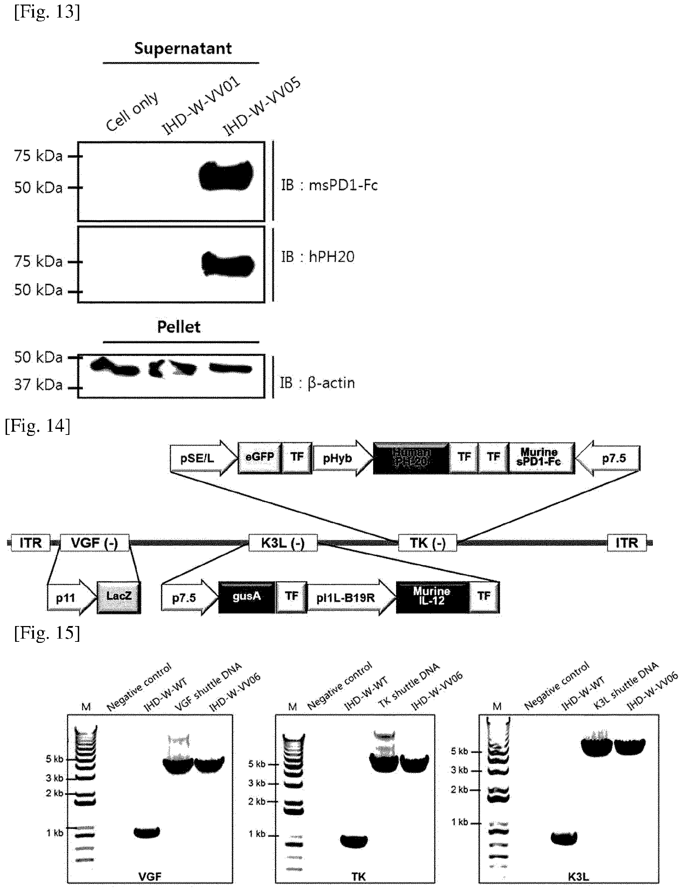

Western blotting was performed to identify that the inserted sPD1-Fc and PH20 genes are expressed, via the recombinant virus, in infected cells. First, HeLa cells were treated with recombinant IHD-W-VV05 at 0.05 MOI for 44 to 52 hours. Then, the medium was replaced with MEM medium containing no fetal bovine serum, and the cells were incubated for 16 to 20 hours. The medium and the cells were separated from each other, and proteins were obtained therefrom, respectively. The prepared proteins were quantified by the Bradford assay method, and then Western blotting was used to identify the protein expression. Here, for the sPD1-Fc gene, anti-mPdcd1 (E-18) (Santacruz, Cat # SC-10299) was used as a primary antibody and anti-Goat was used as a secondary antibody; and for the PH20 gene, anti-hPH20 (LsBio, Cat # LS-C331909) was used as a primary antibody and anti-Rabbit was used as a secondary antibody. The expression results are illustrated in Fig. 13.

**[Table 14]**

| Name | Sequence (5'→3') | SEQ ID NO |
|---|---|---|
| VGF forward | AGGTTCCGTAAGCAAAGAATATAAG | SEQ ID NO: 61 |
| VGF reverse | AGGTAACTAGATTTTACTTTAATATGCCGAA | SEQ ID NO: 62 |
| TK forward | GGAAGGGTCAAACTTAATAAAGGAT | SEQ ID NO: 63 |
| TK reverse | ACCGTGTCGCTGTAACTTACTA | SEQ ID NO: 64 |
| K3L forward | GACGCTGAACACGTTAACGATAGT | SEQ ID NO: 65 |
| K3L reverse | ACTGCGACGAGATACAACCGGA | SEQ ID NO: 66 |

### Example 1.6. Production of IHD-W-VV06 virus

### Example 1.6.1. Production of recombinant IHD-W vaccinia virus in which VGF, TK, and K3L genes are deleted and expression of sPD1-Fc, PH20, and IL-12 genes is induced

Recombinant IHD-W-VV06, in which VGF, TK, and K3L genes are deleted and expression of sPD1-Fc, PH20, and IL-12 genes is induced, was obtained in the same conditions and methods as in Example 1.1.4., except that the IHD-W K3L(IL-12) shuttle plasmid as constructed in Example 1.4.1. and the IHD-W-VV05 as produced in Example 1.5.2. were used; and the gene structure thereof is illustrated in Fig. 14.

### Example 1.6.2. Identification of insertion and expression of sPD1-Fc, PH20, and IL-12 genes in IHD-W-VV06

gDNA of recombinant IHD-W-VV06 as produced in Example 1.6.1. was obtained using the Maxwell purification kit (Cat # AS1330), and the structure of gDNA of the recombinant virus was identified using the primers in Table 15 below. As illustrated in Fig. 15, the results obtained by identifying the DNA structure at each of the VGF, TK, and K3L positions showed that there are changes in PCR fragment sizes at the positions where the genes have been inserted, as compared with a control group.

Western blotting was performed to identify that the inserted sPD1-Fc, PH20, and IL-12 genes are expressed, via the recombinant virus, in infected cells. First, HeLa cells were treated with recombinant IHD-W-VV06 at 0.05 MOI for 44 to 52 hours. Then, the medium was replaced with MEM medium containing no fetal bovine serum, and the cells were incubated for 16 to 20 hours. The medium and the cells were separated from each other, and proteins were obtained therefrom, respectively. The prepared proteins were quantified by the Bradford assay method, and then Western blotting was used to identify the protein expression. Here, for the sPD1-Fc gene, anti-mPdcd1 (E-18) (Santacruz, Cat # SC-10299) was used as a primary antibody and anti-Goat was used as a secondary antibody; for the PH20 gene, anti-hPH20 (LsBio, Cat # LS-C331909) was used as a primary antibody and anti-Rabbit was used as a secondary antibody; and for the IL-12 gene, anti-mIL-12/IL-23 p40 (R&D Systems, Cat # MAB4991) was used as a primary antibody and anti-Rat was used as a secondary antibody. The expression results are illustrated in Fig. 16.

**[Table 15]**

| Name | Sequence (5'→3') | SEQ ID NO |
|---|---|---|
| VGF forward | AGGTTCCGTAAGCAAAGAATATAAG | SEQ ID NO: 67 |
| VGF reverse | AGGTAACTAGATTTTACTTTAATATGCCGAA | SEQ ID NO: 68 |
| TK forward | GGAAGGGTCAAACTTAATAAAGGAT | SEQ ID NO: 69 |
| TK reverse | ACCGTGTCGCTGTAACTTACTA | SEQ ID NO: 70 |
| K3L forward | CGAATAGCCAAACGGCGAGAAG | SEQ ID NO: 71 |
| K3L reverse | ACGGATACATGTGGAGCATCTATTA | SEQ ID NO: 72 |

### Example 2. Production of recombinant WR vaccinia virus

### Example 2.1. Production of WR-VV01 virus

### Example 2.1.1. Construction of recombinant WR vaccinia viral plasmid in which expression of VGF gene is inactivated

Genetic regions that flank VGF gene on both sides in genomic DNA of WR vaccinia virus (ATCC, Cat No. VR-1354) were amplified by PCR, and then respectively inserted into pGEM-T Easy, to construct pGEM-T Easy-VGF-L(WR) and pGEM-T Easy-VGF-R(WR). Information on primers used for the amplification of homologous nucleotide sequences that flank the VGF gene on both sides is shown in Table 16 below.

**[Table 16]**

| Name | Sequence (5'→3') | SEQ ID NO |
|---|---|---|
| VGF-L forward (WR) | CGCAGCTGTGTTATCGATTGATAGTGGTGTCCT | SEQ ID NO: 73 |
| VGF-L reverse (WR) | CTGCAGCGCTAGCACCGCATAATCTGATAGCTGGAAT A | SEQ ID NO: 74 |
| VGF-R forward (WR) | CGGGATCCGTTAATTAAACTCGACGAACTAAACTACC TATAC | SEQ ID NO: 75 |
| VGF-R reverse (WR) | CGATATCGGAAAATGTCTGTTAGTAAATAACCATC | SEQ ID NO: 76 |
| p11 promoter forward | CGGCTAGCTCTAGAAGCGATGCTACGCTAG | SEQ ID NO: 77 |
| p11 promoter reverse | CAAGCTTCGGTTGCCTCGAGGAATTCATTTATAGCATA GAA | SEQ ID NO: 78 |
| LacZ forward | CGCTCGAGGGATCCCGTCGTTTTACAACGTC | SEQ ID NO: 79 |
| LacZ reverse | AAGCTTCTTAATTAAGGATCCCCCCTGCCCGGTTATTA TTATTTTTGACACCAGACCAACT | SEQ ID NO: 80 |

LacZ, whose expression is regulated by p11 promoter, was used as a marker for screening for a virus in which recombination had occurred at a position of the VGF gene. A p11 promoter site in the WR gDNA was amplified by PCR and LacZ gene in pAAV-LacZ (Stratagene, Cat No. 240071-52) was amplified by PCR. Then, the resultants were inserted into pGEM-T Easy and pGEM-T, respectively, to construct pGEM-T Easy-p11 and pGEM-T-LacZ, respectively. Information on primers used for the amplification of the p11 promoter and the LacZ is shown in Table 19.

In order to construct a shuttle plasmid in which the function of the VGF gene is partially deleted, the pGEM-T Easy-VGF-L(WR) was treated with PvuII and PstI, and ligated with a vector obtained by treating pSP72 (Promega, Cat No. P2191) with PvuII and PstI, to construct pSP72-VGF-L(WR). In addition, the pGEM-T Easy-VGF-L-VGF-R(WR) was treated with EcoRV and BamHI, and ligated with a vector obtained by treating the above-constructed pSP72-VGF-L(WR) with EcoRV and BamHI, to obtain pSP72-VGF-L-VGF-R(WR). In order to introduce a LacZ expression cassette, the PGEM-T Easy-p11 was treated with SalI and NheI, and ligated with a vector obtained by treating the pSP72-VGF-L-VGF-R(WR) with SalI and NheI, to construct pSP72-VGF-L-p11-VGF-R(WR). The constructed pSP72-VGF-L-p11-VGF-R(WR) was treated with EcoRI and PacI, and then the above-constructed pGEM-T-LacZ was cut with EcoRI and PacI. The resultants were ligated to complete pSP72-VGF-L-p11-LacZ-VGF-R(WR) (hereinafter referred to as "WR VGF(i) shuttle plasmid") which is a VGF shuttle plasmid.

### Example 2.1.2. Construction of recombinant WR vaccinia viral plasmid in which expression of TK gene is inactivated

In order to obtain genetic regions that flank TK gene on both sides in genomic DNA of WR vaccinia virus, WR gDNA was amplified by PCR, and then the homologous nucleotide sequence fragments located at the left and right sides of the TK gene were respectively inserted into pGEM-T Easy, to construct pGEM-T Easy-TK-L(WR) and pGEM-T Easy-TK-R(WR). Information on primers used for the amplification of homologous nucleotide sequences that flank the TK gene on both sides is shown in Table 17.

**[Table 17]**

| Name | Sequence (5'→3') | SEQ ID NO |
|---|---|---|
| TK-L forward (WR) | AGGTCGACTTGCGATCAATAAATGGATCACAAC | SEQ ID NO: 81 |
| TK-L reverse (WR) | TTAGCTGCAGTATGCGGCCGCAACAATGTCTGGAAA GAACTGTCC | SEQ ID NO: 82 |
| TK-R forward (WR) | CGGAATTCTGTGAGCGTATGGCAA | SEQ ID NO: 83 |
| TK-R reverse (WR) | TCGGGATCCTCAGTCTCATGTTCTCACCGG | SEQ ID NO: 84 |
| p7.5 promoter forward | AGGAAGCTTTCCAAACCCACCCGCTTTTTAT | SEQ ID NO: 85 |
| p7.5 promoter reverse | GAATTCGCACTAGTTCCGATCGCCGTGCAATAAATT AGAATATACCC | SEQ ID NO: 86 |
| EGFP forward | CGCTCGAGATGGTGAGCAAGGGCGAGG | SEQ ID NO: 87 |
| EGFP reverse | TGAGATCTTTACTTGTACAGCTCGTCCATG | SEQ ID NO: 88 |
| Gpt forward | CGACTAGTACACAAGACAGGCTTGCGAG | SEQ ID NO: 89 |
| Gpt reverse | CGGAATTCGGCCCACTCATAAATCCAGTT | SEQ ID NO: 90 |
| pSE/L promoter forward | CGAGCTGCAGATAAAAATTAATTAATTACCCGGGTA CCAGGCCTAGATCTGTCGACTCGAGCTTATTTATATT CCAAAAAAAAAAAATAAAATTTCAATTTTTAAGCTT CGGGATCCGCAA | SEQ ID NO: 91 |
| pSE/L promoter reverse | TTGCGGATCCCGAAGCTTAAAAATTGAAATTTTATTT TTTTTTTTTGGAATATAAATAAGCTCGAGTCGACAGA TCTAGGCCTGGTACCCGGGTAATTAATTAATTTTTAT CTGCAGCTCG | SEQ ID NO: 92 |
| TF forward | ATCGGCGGCCGCTTTTTATCTGCGCGGTTAACCGCC TTTTTATCCATCAGGTGATCTGTTTTTATTGTGGAGC TGCAGCGAT | SEQ ID NO: 93 |
| TF reverse | ATCGCTGCAGCTCCACAATAAAAACAGATCACCTGA TGGATAAAAAGGCGGTTAACCGCGCAGATAAAAAG CGGCCGCCGAT | SEQ ID NO: 94 |

EGFP, whose expression is regulated by pSE/L promoter, and Gpt, whose expression is regulated by p7.5 promoter, were used as markers for screening for a virus in which recombination had occurred at a position of the TK gene. A p7.5 promoter site was amplified by PCR using the WR gDNA as a template, and EGFP gene in pEGFP-N3 (Clontech, Cat No. 6080-1) and Gpt gene in DH5α (Takara, Cat No. 9057) were also amplified by PCR. Then, the resultants were respectively inserted into pGEM-T Easy, to construct pGEM-T Easy-p7.5, pGEM-T Easy-EGFP, and pGEM-T Easy-Gpt, respectively. In addition, pSE/L promoter and TF were constructed through primer annealing. Sequences of primers used in the experiments are shown in Table 17.

The pGEM-T Easy-p7.5 and the annealed pSE/L promoter were respectively treated with BamHI and PstI, and ligated to construct pGEM-T Easy-pSE/L-p7.5. The constructed pGEM-T Easy-pSE/L-p7.5 and pGEM-T Easy-EGFP were respectively treated with BglII and XhoI, and then ligated to construct pGEM-T Easy-EGFP-pSE/L-p7.5.

In order to construct a shuttle plasmid in which the function of the TK gene is partially deleted, the pSP72 was treated with EcoRI and BamHI, and the pGEM-T Easy-TK-R(WR) was treated with EcoRI and BamHI. Then, the resultants were ligated to construct pSP72-TK-R(WR). The constructed pSP72-TK-R(WR) was treated with XhoI and PstI, and ligated with the pGEM-T Easy-TK-L obtained by being treated with SalI and PstI, to construct pSP72-TK-L-TK-R(WR). In order to introduce an EGFP expression cassette, the constructed pSP72-TK-L-TK-R(WR) and pGEM-T Easy-EGFP-pSE/L-p7.5 were respectively treated with EcoRI and PstI, and ligated to construct pSP72-TK-L-EGFP-pSE/L-p7.5-TK-R(WR).

In addition, the constructed pSP72-TK-L-EGFP-pSE/L-p7.5-TK-R(WR) and the annealed TF oligomer were respectively treated with PstI and NotI, and ligated to construct pSP72-TK-L-TF-EGFP-pSE/L-p7.5-TK-R(WR). In order to introduce a Gpt expression cassette, the constructed pSP72-TK-L-TF-EGFP-pSE/L-p7.5-TK-R(WR) and pGEM-T Easy-Gpt were respectively treated with EcoRI and SpeI, and then ligated to finally construct pSP72-TK-L-TF-EGFP-pSE/L-p7.5-Gpt-TK-R(WR) (hereinafter referred to as "WR TK(i) shuttle plasmid") which is a TK shuttle plasmid.

### Example 2.1.3. Construction of recombinant WR vaccinia viral plasmid in which expression of K3L gene is inactivated

A genomic region that flanks K3L gene on the left side and a part of the K3L gene in genomic DNA of WR vaccinia virus were amplified by PCR. Here, the amplification was carried out with the start codon of the K3L gene being placed immediately after the K3L-L sequence, and primers used for the amplification of the homologous sequence on the left side of the K3L gene are shown in Table 18. Here, a K3L-L-K3Li(WR) fragment which was amplified and includes a part that excludes and follows the start codon of the K3L gene was obtained and then ligated with a pGEM-T Easy vector, to construct pGEM-T Easy-K3L-L-K3Li(WR).

**[Table 18]**

| Name | Sequence (5'→3') | SEQ ID NO |
|---|---|---|
| K3L-Li forward | TGTACGTATATTTAGATGTTTTCAGCT | SEQ ID NO: 95 |
| K3L-Li reverse | ATAAGCTTCTTGCATTTTGTTATTCGT | SEQ ID NO: 96 |
| K3L-Ri forward | CGCAGATAAAAACATATCCTTGTTAAC | SEQ ID NO: 97 |
| K3L-Ri reverse | GTTAACAAGGATATGTTTTTATCTGCG | SEQ ID NO: 98 |

The IHD-W K3L shuttle plasmid as constructed in Example 1.1.3 and the pGEM-T Easy-K3L-L-K3Li(WR) were treated with SnaBI and HindIII, and ligated to construct pSP72-K3L-L-K3Li-p7.5-DsRed-TF-K3L-R(WR). In order to introduce the start codon of K3L into the constructed pSP72-K3L-L-K3Li-p7.5-DsRed-TF-K3L-R(WR), a point mutation was performed using primers as shown in Table 18 above, so that pSP72-K3L-L-K3Li-p7.5-DsRed-TF-K3L_{ATG}-K3L-R(WR) (hereinafter referred to as "WR K3L(i) shuttle plasmid") was finally constructed.

### Example 2.1.4. Production of recombinant WR vaccinia virus in which expression of VGF and TK genes is inactivated

First, recombinant WR vaccinia virus Vi in which expression of VGF gene is inactivated was obtained in the same conditions and methods as in Example 1.1.4., except that the WR VGF(i) shuttle plasmid and WR wild-type virus were used. Thereafter, recombinant WR vaccinia virus ViTi in which expression of VGF and TK genes is inactivated was obtained in the same methods as above, except that the WR TK(i) shuttle plasmid and the recombinant WR vaccinia virus Vi were used.

### Example 2.1.5. Production of WR-VV01 virus

First, recombinant WR vaccinia virus WR-VV01 in which expression of VGF, TK, and K3L genes is inactivated was obtained in the same conditions and methods as in Example 1.1.4., except that the WR K3L(i) shuttle plasmid and the recombinant WR vaccinia virus ViTi were used; and the gene structure thereof is illustrated in Fig. 17.

### Example 2.2. Production of WR-VV06 virus

### Example 2.2.1. Construction of recombinant WR vaccinia viral plasmid in which expression of TK gene is inactivated and expression of sPD1-Fc and PH20 genes is induced

First, the WR TK(i) shuttle plasmid as constructed in Example 2.1.2. and the IHD-W TK(sPD1-Fc) shuttle plasmid as constructed in Example 1.2.1. were respectively treated with BamHI and EcoRI, and then ligated to construct PSP72-TK-L-TF-TF-EGFP-pSE/L-TF-sPD1-Fc-p7.5-TK-R(WR) (hereinafter referred to as "WR TKi(sPD1-Fc) shuttle plasmid"). Thereafter, the thus constructed plasmid and the IHD-W TK(PH20) shuttle plasmid as constructed in Example 1.3.1. were respectively treated with BamHI and PacI, and then ligated to finally construct pSP72-TK-L-TF-pSE/L-EGFP-TF-pHyb-PH20-TF-TF-sPD1-Fc-p7.5-TK-R(WR) (hereinafter referred to as "WR TKi(sPD1-Fc + PH20) shuttle plasmid").

### Example 2.2.2. Construction of recombinant WR vaccinia viral plasmid in which expression of K3L gene is inactivated and expression of IL-12 gene is induced

In order to construct a gene expression cassette inside the WR K3Li shuttle plasmid, the WR K3Li shuttle plasmid as constructed in Example 2.1.3. and the IHD-W K3L(IL-12) shuttle plasmid as constructed in Example 1.4.1. were treated with Sa1I, and ligated to finally construct pSP72 K3L-L-p7.5-DsRed-TF-pI1L-B19R-IL-12-TF-TF_K3L-R(WR) (hereinafter referred to as "WR K3Li(IL-12) shuttle plasmid").

### Example 2.2.3. Production of WR-VV06 virus

First, recombinant WR vaccinia virus ViKi in which expression of VGF and K3L genes is inactivated was obtained in the same conditions and methods as in Example 1.1.4., except that the WR K3Li shuttle plasmid as constructed in Example 2.1.3. and the recombinant WR vaccinia virus Vi as constructed in Example 2.1.4. were used.

Recombinant WR vaccinia virus WR-VV05, in which expression of VGF, TK, and K3L genes is inactivated and expression of sPD1-Fc and PH20 genes is induced, was obtained in the same conditions and methods as in Example 1.1.4., except that the WR TKi(sPD1-Fc + PH20) shuttle plasmid as constructed in Example 2.2.1. and the recombinant WR vaccinia virus ViKi were used.

Recombinant WR-VV06, in which expression of VGF, TK, and K3L genes is inactivated and expression of sPD1-Fc, PH20, and IL-12 genes is induced, was obtained in the same conditions and methods as in Example 1.1.4., except that the WR K3Li(IL-12) shuttle plasmid as constructed in Example 2.2.2. and the recombinant WR-VV05 were used; and the gene structure thereof is illustrated in Fig. 18.

### Example 2.2.4. Identification of insertion and expression of sPD1-Fc, PH20, and IL-12 genes in WR-VV06

gDNA of the recombinant WR-VV06 as produced in Example 2.2.2. was obtained using the Maxwell purification kit (Cat # AS1330), and the structure of gDNA of the recombinant virus was identified using the primers in Table 19 below. As illustrated in Fig. 19, the results obtained by identifying the DNA structure at each of the VGF, TK, and K3L positions showed that there are changes in PCR fragment sizes at the positions where the genes have been inserted, as compared with a control group.

Western blotting was performed to identify that the inserted sPD1-Fc and PH20 genes are expressed, via the recombinant virus, in infected cells. First, HeLa cells were treated with the recombinant WR-VV06 at 0.05 MOI for 44 to 52 hours. Then, the medium was replaced with MEM medium containing no fetal bovine serum, and the cells were incubated for 16 to 20 hours. The medium and the cells were separated from each other, and proteins were obtained therefrom, respectively. The prepared proteins were quantified by the Bradford assay method, and then Western blotting was used to identify the protein expression. Here, for the sPD1-Fc gene, anti-mPdcd1 (E-18) (Santacruz, Cat # SC-10299) was used as a primary antibody and anti-Goat was used as a secondary antibody; and for the PH20 gene, anti-hPH20 (LsBio, Cat # LS-C331909) was used as a primary antibody and anti-Rabbit was used as a secondary antibody. The expression results are illustrated in Fig. 20a.

In addition, ELISA was performed to identify that the inserted IL-12 gene is expressed, via the recombinant virus, in infected cells. First, HeLa cells were treated with the recombinant WR-VV06 at 0.05 MOI. After 44 to 52 hours, only the medium was separated and expressed proteins were obtained therefrom. For the prepared proteins, the Mouse IL-12 p70 Quantikine ELISA kit (R&D Systems, Cat # M1270) was used to identify the protein expression level. Here, the protein stock solution was diluted to 1/5,000 and used. The measurement results are illustrated in Fig. 20b.

**[Table 19]**

| Name | Sequence (5'→3') | SEQ ID NO |
|---|---|---|
| VGF forward | AGGTTCCGTAAGCAAAGAATATAAG | SEQ ID NO: 99 |
| VGF reverse | AGGTAACTAGATTTTACTTTAATATGCCGAA | SEQ ID NO: 100 |
| TK forward | GGAAGGGTCAAACTTAATAAAGGAT | SEQ ID NO: 101 |
| TK reverse | ACCGTGTCGCTGTAACTTACTA | SEQ ID NO: 102 |
| K3L forward | CGAATAGCCAAACGGCGAGAAG | SEQ ID NO: 103 |
| K3L reverse | ACGGATACATGTGGAGCATCTATTA | SEQ ID NO: 104 |

### Example 3. Production of recombinant Lister vaccinia virus

### Example 3.1. Production of Lister-VV01 virus

### Example 3.1.1. Construction of recombinant Lister vaccinia viral plasmid in which VGF gene is deleted

First, genetic regions that flank VGF gene on both sides in genomic DNA of Lister vaccinia virus (ATCC, VR-1549) were amplified by PCR, and then respectively inserted into pGEM-T Easy, to construct pGEM-T Easy-VGF-L(Lister) and pGEM-T Easy-VGF-R(Lister). Information on primers used for the amplification of homologous nucleotide sequences that flank the VGF gene on both sides is shown in Table 20 below.

The pSP72 vector and the pGEM-T Easy-VGF-L(Lister) were respectively treated with HindIII and SalI, and ligated to construct pSP72-VGF-L(Lister). In addition, the pSP72-VGF-L(Lister) and the pGEM-T Easy-VGF-R(Lister) were respectively treated with PacI and KpnI, and ligated to construct pSP72-VGF-L-VGF-R(Lister).

The constructed pSP72-VGF-L-VGF-R(Lister) vector and the IHD-W VGF shuttle plasmid as constructed in Example 1.1.1. were respectively treated with NheI and PacI, and ligated to finally construct pSP72-VGF-L-p11-LacZ-VGF-R(Lister) (hereinafter referred to as "Lister VGF shuttle plasmid").

**[Table 20]**

| Name | Sequence (5' → 3') | SEQ ID NO |
|---|---|---|
| VGF-L forward (Lister) | ATGAAGCTTTAAAACTTGTCTGTATATGTAAGATGT TT | SEQ ID NO: 105 |
| VGF-L reverse (Lister) | ACCCGGGTTTAATTAACATGCTAGCGTGTAAATAAT ATAAAAATAACAATACAATATTG | SEQ ID NO: 106 |
| VGF-R forward (Lister) | GCTTAATTAAGGCGGCCGCTTTTATAAATTTTTTT ATGAGTATTTTTACAAAAT | SEQ ID NO: 107 |
| VGF-R reverse (Lister) | TCCCGGGCATCGATAGTACAATAACTTTTATGAAAT | SEQ ID NO: 108 |

### Example 3.1.2. Construction of recombinant Lister vaccinia viral plasmid in which TK gene is deleted

Genetic regions that flank TK gene on both sides in genomic DNA of Lister vaccinia virus (ATCC, VR-1549) were amplified by PCR, and then respectively inserted into pGEM-T Easy, to construct pGEM-T Easy-TK-L(Lister) and pGEM-T Easy-TK-R(Lister). Information on primers used for the amplification of homologous nucleotide sequences that flank the TK gene on both sides is shown in Table 21 below.

The pGEM-T Easy-TK-L(Lister) and the pGEM-T Easy-TK-R(Lister) were respectively treated with BamHI and XmaI, and ligated to construct pGEM-T Easy-TK-L-TK-R(Lister). In addition, the pSP72 vector and the pGEM-T Easy-TK-L-TK-R(Lister) were respectively treated with SpeI and XmaI, and ligated to construct pSP72-TK-L-TK-R(Lister).

The constructed pSP72-TK-L-TK-R(Lister) vector and the IHD-W TK shuttle plasmid as constructed in Example 1.1.2. were respectively treated with NotI and SalI, and ligated to finally construct pSP72-TK-L-TF-pSE/L-EGFP-TK-R(Lister) (hereinafter, "Lister TK shuttle plasmid").

**[Table 21]**

| Name | Sequence (5' → 3') | SEQ ID NO |
|---|---|---|
| TK-L forward (Lister) | CCCCGGGATAGGATCCATAGTCGACTTTAATTAATGCG GCCGCATGACAATAAAGAATTAATTATTGTTCAC | SEQ ID NO: 109 |
| TK-L reverse (Lister) | AGACTAGTCCCTCTTCAAGAACCCATTAG | SEQ ID NO: 110 |
| TK-R forward (Lister) | CCCCGGGGCTTTAGTAGTAGGAAATGTTTTATTG | SEQ ID NO: 111 |
| TK-R reverse (Lister) | TAAGGATCCTCTGCTAGCTATTATATTTTTTATCTAAAA AACTAAAAATAAACAT | SEQ ID NO: 112 |

### Example 3.1.3. Construction of recombinant Lister vaccinia viral plasmid in which K3L gene is deleted

The IHD-W K3L shuttle plasmid as constructed in Example 1.1.3. was treated with BglII and EcoRV, and a gene (R-arm) located on the right side of the K3L gene in genomic DNA of Lister vaccinia virus (ATCC, VR-1549) was amplified by PCR. Then, insertion thereof was performed using an infusion cloning method, to construct pSP72-p7.5-DsRed-TF-TF-K3L-R(Lister).

The constructed pSP72-p7.5-DsRed-TF-TF-K3L-R(Lister) was treated with XhoI and HindIII, and a gene (L-arm) located on the left side of the K3L gene in genomic DNA of Lister vaccinia virus was amplified by PCR. Then, insertion thereof was performed using an infusion cloning method, to finally construct pSP72-K3L-L-p7.5-DsRed-TF-TF-K3L-R(Lister) (hereinafter referred to as "Lister K3L shuttle plasmid"). Sequences of primers used in the infusion cloning process are shown in Table 22 below.

**[Table 22]**

| Name | Sequence (5'→3') | SEQ ID NO |
|---|---|---|
| K3L-L forward (Lister) | CACTATAGAACTCGAGCATATGTTTAAACGACGCA TTATCTG | SEQ ID NO: 113 |
| K3L-L reverse (Lister) | TGGGTTTGGAAAGCTTTTTTTATACCGAACATAAA AATAAGG | SEQ ID NO: 114 |
| K3L-R forward (Lister) | CGCAGATAAAAAGATATCCTTGTTAACGGGCTCGT AAATTG | SEQ ID NO: 115 |
| K3L-R reverse (Lister) | GGAGACCGGCAGATCTTGATAATACACATATTTATT TAGGAAGCG | SEQ ID NO: 116 |

### Example 3.1.4. Production of recombinant Lister vaccinia virus in which VGF gene is deleted

Recombinant Lister vaccinia virus V in which VGF gene is deleted was obtained in the same conditions and methods as in Example 1.1.4., except that Lister wild-type vaccinia virus and the Lister VGF shuttle plasmid as constructed in Example 3.1.1. were used.

### Example 3.1.5. Production of recombinant Lister vaccinia virus in which VGF and TK genes are deleted

Recombinant Lister vaccinia virus VT in which VGF and TK genes are deleted was obtained in the same conditions and methods as in Example 1.1.4., except that the recombinant Lister vaccinia virus V as produced in Example 3.1.4. and the Lister TK shuttle plasmid as constructed in Example 3.1.2. were used.

### Example 3.1.6. Production of Lister-VV01 virus

Recombinant Lister-VV01 in which VGF, TK, and K3L genes are deleted was obtained in the same conditions and methods as in Example 1.1.4., except that the recombinant Lister vaccinia virus VT as produced in Example 3.1.5. and the Lister K3L shuttle plasmid as constructed in Example 3.1.3. were used; and the gene structure thereof is illustrated in Fig. 21.

### Example 3.2. Production of Lister-VV06 virus

### Example 3.2.1. Construction of recombinant Lister vaccinia viral plasmid in which TK gene is deleted and expression of sPD1-Fc and PH20 genes is induced

The plasmid as constructed in Example 3.1.2. and the IHD-W TK(sPD1-Fc + PH20) shuttle plasmid as constructed in Example 1.5.1 were respectively treated with NotI and NheI, and ligated to finally construct pSP72-TK-L-TF-pSE/L-EGFP-pHyb-PH20-TF-TF-sPD1-Fc-p7.5-TK-R(Lister) (hereinafter referred to as "Lister TK(sPD1-Fc + PH20) shuttle plasmid").

### Example 3.2.2. Construction of recombinant Lister vaccinia viral plasmid in which K3L gene is deleted and expression of IL-12 gene is induced

In order to construct a gene expression cassette inside the Lister K3L shuttle plasmid, the Lister K3L shuttle plasmid as constructed in Example 3.1.3. and the IHD-W K3L(IL-12) shuttle plasmid as constructed in Example 1.4.1. were treated with SalI, and ligated to finally construct pSP72 K3L-L-p7.5-DsRed-TF-pI1L-B19R-IL-12-TF-TF-K3L-R(Lister) (hereinafter, "Lister K3L(IL-12) shuttle plasmid").

### Example 3.2.3. Production of Lister-VV06 virus

First, recombinant Lister vaccinia virus VK in which VGF and K3L genes are deleted was obtained in the same conditions and methods as in Example 1.1.4., except that the recombinant Lister vaccinia virus V as produced in Example 3.1.4. and the Lister K3L shuttle plasmid as constructed in Example 3.1.3. were used.

Recombinant Lister vaccinia virus Lister-VV05, in which VGF, TK, and K3L genes are deleted and expression of sPD1-Fc and PH20 genes is induced, was obtained in the same conditions and methods as in Example 1.1.4., except that the Lister TK(sPD1-Fc + PH20) shuttle plasmid as constructed in Example 3.2.1. and the recombinant Lister vaccinia virus VK were used.

Recombinant Lister-VV06 in which VGF, TK, and K3L genes are deleted and expression of sPD1-Fc, PH20, and IL-12 genes is induced was obtained in the same conditions and methods as in Example 1.1.4., except that the Lister K3L(IL-12) shuttle plasmid as constructed in Example 3.2.2. and the recombinant Lister-VV05 were used; and the gene structure thereof is illustrated in Fig. 22.

### Example 3.2.4. Identification of insertion and expression of sPD1-Fc, PH20, and IL-12 genes in Lister-VV06

gDNA of recombinant Lister-VV06 as produced in Example 3.2.3. was obtained using the Maxwell viral total nucleic acid purification kit (Promega, Cat # AS1330), and the structure of gDNA of the recombinant virus, Lister-VV06, was identified using the primers in Table 23 below. As illustrated in Fig. 23, the results obtained by identifying the DNA structure at each of the VGF, TK, and K3L positions showed that there are changes in PCR fragment sizes at the positions where the genes have been inserted, as compared with a control group. Western blotting was performed to identify that the inserted sPD1-Fc gene is expressed, via the recombinant virus, in infected cells. First, HeLa cells were treated with recombinant Lister-VV06 at 0.05 MOI for 44 to 52 hours. Then, the medium was replaced with MEM medium containing no fetal bovine serum, and the cells were incubated for 16 to 20 hours. The medium and the cells were separated from each other, and proteins were obtained therefrom, respectively.

The prepared proteins were quantified by the Bradford assay method, and then Western blotting was used to identify the protein expression. Here, anti-mPdcd1 (E-18) (Santacruz, Cat # SC-10299) was used as a primary antibody and anti-Goat was used as a secondary antibody. In addition, Western blotting was performed to identify that the inserted PH-20 gene is expressed, via the recombinant virus, in infected cells. Here, anti-hPH20 (LsBio, Cat # LS-C331909) was used as a primary antibody and anti-Rabbit was used as a secondary antibody. The expression results are illustrated in Fig. 24a. In addition, ELISA was performed to identify that the inserted IL-12 gene is expressed, via the recombinant virus, in infected cells. First, HeLa cells were infected with recombinant Lister-VV06 at 0.05 MOI. After 44 to 52 hours, only the medium was separated and expressed proteins were obtained therefrom. For the prepared proteins, the Mouse IL-12 p70 Quantikine ELISA kit (R&D Systems, Cat # M1270) was used to identify the protein expression level. Here, the protein stock solution was diluted to 1/5,000 and used. The measurement results are illustrated in Fig. 24b.

**[Table 23]**

| Name | Sequence (5' → 3') | SEQ ID NO |
|---|---|---|
| VGF forward | AGGTTCCGTAAGCAAAGAATATAAG | SEQ ID NO: 117 |
| VGF reverse | AGGTAACTAGATTTTACTTTAATATGCCGAA | SEQ ID NO: 118 |
| TK forward | GGAAGGGTCAAACTTAATAAAGGAT | SEQ ID NO: 119 |
| TK reverse | ACCGTGTCGCTGTAACTTACTA | SEQ ID NO: 120 |
| K3L forward | GACGCTGAACACGCTAACGATAGT | SEQ ID NO: 121 |
| K3L reverse | ACTGCGACGAGATACAACCGGA | SEQ ID NO: 122 |

### II. Determination of tumor cell-killing ability of recombinant vaccinia virus in vivo

### Experimental Example 1. Determination of tumor suppression efficacy, in animal model, of recombinant IHD-W strain vaccinia virus in which VGF, TK, and K3L genes are deleted and expression of sPD1-Fc or PH20 or IL-12 gene is induced

### Experimental Example 1.1. Determination of tumor suppression efficacy, in animal model, of IHD-W-VV02, IHD-W-VV03, and IHD-W-VV05 viruses

Comparison of anti-tumor effects among the recombinant IHD-W-VV02, IHD-W-VV03, and IHD-W-VV05 as produced in Example 1 was made in a mouse model.

### Colorectal tumor model

First, the colorectal cancer cell line CT26.WT was prepared by being incubated in RPMI medium containing 10% fetal bovine serum. In a case where the cells that were being incubated in an incubator under a condition of 37°C and 5% CO₂ occupied 70% to 80% of a dish, the cells were prepared for cancer cell inoculation. The prepared cancer cells were centrifuged at 1,500 rpm for 5 minutes at 4°C to remove all supernatant, and the cells were prepared by adding an excipient (RPMI medium) thereto. The 1×10⁶ cells thus prepared were injected subcutaneously in the right flank of BALB/c mice (BALB/cAnHsd; KOATECH, Korea) to prepare a mouse colorectal tumor model. After one week, in a case where the tumor volume grew to about 70 to 100 mm3, the prepared mouse model was divided into groups to be administered with PBS, IHD-W-VV02, IHD-W-VV03, and IHD-W-VV05 with 4 mice per group, and then 50 ul of PBS (Welgene, Cat No. LB001-02) or each of the viruses at 1×107 TCID50/50 ul was administered once into the tumor. The results obtained by measuring the tumor volume after the virus administration are illustrated in Fig. 25.

As illustrated in Fig. 25, the results obtained by measuring the tumor size on day 14 after the virus treatment showed that the IHD-W-VV02-administered group, the IHD-W-VV03-administered group, and the IHD-W-VV05-administered group exhibit a smaller tumor volume than that of the PBS-administered group. It was identified that in a case where the tumor volume is compared among the virus-administered groups, the IHD-W-VV05-administered group exhibits a smaller tumor volume than that of the IHD-W-VV02-administered group or the IHD-W-VV03-administered group.

### Experimental Example 1.2. Determination of tumor suppression efficacy, in animal model, of IHD-W-VV01 and IHD-W-VV05 viruses

Comparison of anti-tumor effects between the recombinant IHD-W-VV01 and IHD-W-VV05 as produced in Example 1 was made in a mouse model.

### Skin tumor model

Next, the skin cancer cell line B16F10 was prepared by being incubated in DMEM medium containing 10% fetal bovine serum. In a case where the cells that were being incubated in an incubator under a condition of 37°C and 5% CO₂ occupied 70% to 80% of a dish, the cells were prepared for cancer cell inoculation. The prepared cancer cells were centrifuged at 1,500 rpm for 5 minutes at 4°C to remove all supernatant, and the cells were prepared by adding an excipient (DMEM medium) thereto. The 5×10⁵ cells thus prepared were injected subcutaneously in the right flank of C57BL/6N mice (C57BL/6NHsd; KOATECH, Korea) to prepare a mouse skin tumor model. After one week, in a case where the tumor volume grew to about 70 to 100 mm³, the prepared mouse model was divided into groups to be administered with PBS, IHD-W-VV01, and IHD-W-VV05 with 6 mice per group, and then 50 ul of PBS (Welgene, Cat No. LB001-02) or each of the viruses at 1×10⁶ TCID₅₀/50 ul was administered once into the tumor. The results obtained by measuring the tumor volume after the virus administration are illustrated in Fig. 26.

As illustrated in Fig. 26, the results obtained by measuring the tumor volume on day 12 after the virus treatment showed that the IHD-W-VV05-administered group exhibits a smaller tumor volume than that of the IHD-W-VV01-administered group. In the PBS-administered group, the tumor volume had already reached an average of 2,000 mm³ before day 12 after the virus administration. Thus, the PBS-administered group was euthanized and the data after 12 days were excluded from the analysis.

### Lung tumor model

Next, the lung cancer cell line LLC1 was incubated in an incubator under a condition of 37°C and 5% CO₂ using DMEM medium composed of 10% fetal bovine serum, 2 mM L-glutamine, and 1% antibiotics-antimycotics (GIBCO, Cat No. 15240-062). Three passages were performed, and then the cells were harvested in a case where the cells occupied 70% to 80% of the culture plate during the last passage. The supernatant of the harvested cells was removed, and an excipient (DMEM) was added thereto so that the cells are prepared to have a concentration of 1×10⁷/mL. A cell suspension containing the 1×10⁶ cells thus prepared was injected subcutaneously in the right flank of C57BL/6N mice (C57BL/6NHsd; KOATECH, Korea) to prepare a mouse lung tumor model. After one week, in a case where the tumor volume grew to about 70 to 100 mm³, the prepared mouse model was divided into groups to be administered with PBS, IHD-W-VV01, and IHD-W-VV05 with 6 mice per group. Then, 50 ul of PBS (Welgene, Cat No. LB001-02) or each of the viruses at 1×10⁶ TCID₅₀/50 ul was administered once into the tumor. The results obtained by measuring the tumor volume after the virus administration are illustrated in Fig. 27.

As illustrated in Fig. 27, the results obtained by measuring the tumor size on day 14 after the virus treatment showed that the IHD-W-VV01-administered group and the IHD-W-VV05-administered group exhibit a smaller tumor volume than that of the PBS-administered group. It was identified that in a case where the tumor volume is compared among the virus-administered groups, the IHD-W-VV05-administered group exhibits a smaller tumor volume than that of the IHD-W-VV01-administered group.

### Experimental Example 1.3. Determination of tumor suppression efficacy, in animal model, of IHD-W-VV02, IHD-W-VV03, IHD-W-VV04, and IHD-W-VV06 viruses

Comparison of anti-tumor effects among the recombinant IHD-W-VV02, IHD-W-VV03, IHD-W-VV04 and IHD-W-VV06 as produced in Example 1 was made in a mouse model.

### Colorectal tumor model

First, the colorectal cancer cell line CT26.WT was prepared by being incubated in RPMI medium containing 10% fetal bovine serum. In a case where the cells that were being incubated in an incubator under a condition of 37°C and 5% CO₂ occupied 70% to 80% of a dish, the cells were prepared for cancer cell inoculation. The prepared cancer cells were centrifuged at 1,500 rpm for 5 minutes at 4°C to remove all supernatant, and the cells were prepared by adding an excipient (RPMI medium) thereto. The 1×10⁶ cells thus prepared were injected subcutaneously in the right flank of BALB/c mice (BALB/cAnHsd; KOATECH, Korea) to prepare a mouse colorectal tumor model. After one week, in a case where the tumor volume grew to about 70 to 100 mm³, the prepared mouse model was divided into groups to be administered with PBS, IHD-W-VV02, IHD-W-VV03, IHD-W-VV04, and IHD-W-VV06 with 4 mice per group, and then 50 ul of PBS (Welgene, Cat No. LB001-02) or each of the viruses at 1×10⁷ TCID₅₀/50 ul was administered once into the tumor. The results obtained by measuring the tumor volume after the virus administration are illustrated in Fig. 28.

As illustrated in Fig. 28, the results obtained by measuring the tumor volume on day 14 after the virus treatment showed that the IHD-W-VV02-administered group, the IHD-W-VV03-administered group, the IHD-W-VV04-administered group, and the IHD-W-VV06-administered group exhibit a smaller tumor volume than that of the PBS-administered group. It was identified that when the tumor volume is compared among the virus-administered groups, the IHD-W-VV06-administered group exhibits a smaller tumor volume than that of the IHD-W-VV02-administered group, the IHD-W-VV03-administered group, or the IHD-W-VV04-administered group.

### Lung tumor model

Next, the lung cancer cell line LLC1 was incubated in an incubator under a condition of 37°C and 5% CO₂ using DMEM medium composed of 10% fetal bovine serum, 2 mM L-glutamine, and 1% antibiotics-antimycotics (GIBCO, Cat No. 15240-062). Three passages were performed, and then the cells were harvested in a case where the cells occupied 70% to 80% of the culture plate during the last passage. The supernatant of the harvested cells was removed, and an excipient (DMEM) was added thereto so that the cells are prepared to have a concentration of 1×10⁷/mL. A cell suspension containing the 1×10⁶ cells thus prepared was injected subcutaneously in the right flank of C57BL/6N mice (C57BL/6NHsd; KOATECH, Korea) to prepare a mouse lung tumor model. After one week, in a case where the tumor volume grew to about 70 to 100 mm³, the prepared mouse model was divided into groups to be administered with IHD-W-VV02, IHD-W-VV03, IHD-W-VV04, and IHD-W-VV06 with 6 mice per group. Then, 50 ul of PBS (Welgene, Cat No. LB001-02) or each of the viruses at 1×10⁶ TCID₅₀/50 ul was administered once into the tumor. The results obtained by measuring the tumor volume after the virus administration are illustrated in Fig. 29.

As illustrated in Fig. 29, the results obtained by measuring the tumor size on day 14 after the virus treatment showed that the IHD-W-VV02-administered group, the IHD-W-VV03-administered group, the IHD-W-VV04-administered group, and the IHD-W-VV06-administered group exhibit a smaller tumor volume than that of the PBS-administered group. It was identified that when the tumor volume is compared among the virus-administered groups, the IHD-W-VV06-administered group exhibits a smaller tumor volume than that of the IHD-W-VV02-administered group, the IHD-W-VV03-administered group, or the IHD-W-VV04-administered group.

### Experimental Example 1.4. Determination of tumor suppression efficacy, in animal model, of IHD-W-VV05 and IHD-W-VV06 viruses

Comparison of anti-tumor effects between the recombinant IHD-W-VV05 and IHD-W-VV06 as produced in Example 1 was made in a mouse model.

### Colorectal tumor model

First, the colorectal cancer cell line CT26.WT was prepared by being incubated in RPMI medium containing 10% fetal bovine serum. When the cells that were being incubated in an incubator under a condition of 37°C and 5% CO₂ occupied 70% to 80% of a dish, the cells were prepared for cancer cell inoculation. The prepared cancer cells were centrifuged at 1,500 rpm for 5 minutes at 4°C to remove all supernatant, and the cells were prepared by adding an excipient (RPMI medium) thereto. The 1×10⁶ cells thus prepared were injected subcutaneously in the right flank of BALB/c mice (BALB/cAnHsd; KOATECH, Korea) to prepare a mouse colorectal tumor model. After one week, when the tumor volume grew to about 70 to 100 mm³, the prepared mouse model was divided into groups to be administered with PBS, IHD-W-VV05, and IHD-W-VV06 with 4 mice per group, and then 50 ul of PBS (Welgene, Cat No. LB001-02) or each of the viruses at 1×10⁷ TCID₅₀/50 ul was administered once into the tumor. The results obtained by measuring the tumor volume after the virus administration are illustrated in Fig. 30.

As illustrated in Fig. 30, the results obtained by measuring the tumor volume on day 14 after the virus treatment showed that the IHD-W-VV05-administered group and the IHD-W-VV06-administered group exhibit a smaller tumor volume than that of the PBS-administered group. It was identified that when the tumor volume is compared among the virus-administered groups, the IHD-W-VV06-administered group exhibits a smaller tumor volume than that of the IHD-W-VV05-administered group.

### Skin tumor model

Next, the skin cancer cell line B16F10 was prepared by being incubated in DMEM medium containing 10% fetal bovine serum. When the cells that were being incubated in an incubator under a condition of 37°C and 5% CO₂ occupied 70% to 80% of a dish, the cells were prepared for cancer cell inoculation. The prepared cancer cells were centrifuged at 1,500 rpm for 5 minutes at 4°C to remove all supernatant, and the cells were prepared by adding an excipient (DMEM medium) thereto. The 5×10⁵ cells thus prepared were injected subcutaneously in the right flank of C57BL/6N mice (C57BL/6NHsd; KOATECH, Korea) to prepare a mouse skin tumor model. After one week, when the tumor volume grew to about 70 to 100 mm³, the prepared mouse model was divided into groups to be administered with PBS, IHD-W-VV05, and IHD-W-VV06 with 6 mice per group, and then 50 ul of PBS (Welgene, Cat No. LB001-02) or each of the viruses at 1×10⁶ TCID₅₀/50 ul was administered once into the tumor. The results obtained by measuring the tumor volume after the virus administration are illustrated in Fig. 31.

As illustrated in Fig. 31, the results obtained by measuring the tumor volume on day 12 after the virus treatment showed that the IHD-W-VV06-administered group exhibits a smaller tumor volume than that of the IHD-W-VV05-administered group. In the PBS-administered group, the tumor volume had already reached an average of 2,000 mm³ before day 12 after the virus administration. Thus, the PBS-administered group was euthanized and the data after 12 days were excluded from the analysis.

### Lung tumor model

Next, the lung cancer cell line LLC1 was incubated in an incubator under a condition of 37°C and 5% CO₂ using DMEM medium composed of 10% fetal bovine serum, 2 mM L-glutamine, and 1% antibiotics-antimycotics (GIBCO, Cat No. 15240-062). Three passages were performed, and then the cells were harvested when the cells occupied 70% to 80% of the culture plate during the last passage. The supernatant of the harvested cells was removed, and an excipient (DMEM) was added thereto so that the cells are prepared to have a concentration of 1×10⁷/mL. A cell suspension containing the 1×10⁶ cells thus prepared was injected subcutaneously in the right flank of C57BL/6N mice (C57BL/6NHsd; KOATECH, Korea) to prepare a mouse lung tumor model. After one week, when the tumor volume grew to about 70 to 100 mm³, the prepared mouse model was divided into groups to be administered with PBS, IHD-W-VV05, and IHD-W-VV06 with 6 mice per group. Then, 50 ul of PBS (Welgene, Cat No. LB001-02) or each of the viruses at 1×10⁶ TCID₅₀/50 ul was administered once into the tumor. The results obtained by measuring the tumor volume after the virus administration are illustrated in Fig. 32.

As illustrated in Fig. 32, the results obtained by measuring the tumor volume on day 14 after the virus treatment showed that the IHD-W-VV05-administered group and the IHD-W-VV06-administered group exhibit a smaller tumor volume than that of the PBS-administered group. It was identified that when the tumor volume is compared among the virus-administered groups, the IHD-W-VV06-administered group exhibits a smaller tumor volume than that of the IHD-W-VV05-administered group.

### Experimental Example 2. Determination of tumor suppression efficacy, in animal model, of various recombinant vaccinia viruses in which VGF, TK, and K3L genes are deleted or expression thereof is inactivated and expression of sPD1-Fc or PH20 or IL-12 gene is induced

### Experimental Example 2.1. Determination of tumor suppression efficacy, in animal model, of IHD-W-VV01 and IHD-W-VV06 viruses

Comparison of anti-tumor effects between the recombinant IHD-W-VV01 and IHD-W-VV06 as produced in Example 1 was made in a mouse model.

### Skin tumor model

Next, the skin cancer cell line B16F10 was prepared by being incubated in DMEM medium containing 10% fetal bovine serum. When the cells that were being incubated in an incubator under a condition of 37°C and 5% CO₂ occupied 70% to 80% of a dish, the cells were prepared for cancer cell inoculation. The prepared cancer cells were centrifuged at 1,500 rpm for 5 minutes at 4°C to remove all supernatant, and the cells were prepared by adding an excipient (DMEM medium) thereto. The 5×10⁵ cells thus prepared were injected subcutaneously in the right flank of C57BL/6N mice (C57BL/6NHsd; KOATECH, Korea) to prepare a mouse skin tumor model. After one week, when the tumor volume grew to about 70 to 100 mm³, the prepared mouse model was divided into groups to be administered with PBS, IHD-W-VV01, and IHD-W-VV06 with 4 mice per group, and then 50 ul of PBS (Welgene, Cat No. LB001-02) or each of the viruses at 1×10⁶ TCID₅₀/50 ul was administered once into the tumor. The results obtained by measuring the tumor volume after the virus administration are illustrated in Fig. 33.

As illustrated in Fig. 33, the results obtained by measuring the tumor volume on day 14 after the virus treatment showed that the IHD-W-VV01-administered group exhibits a smaller tumor volume than that of the IHD-W-VV06-administered group. In the PBS-administered group, the tumor volume had already reached an average of 2,000 mm³ before day 12 after the virus administration. Thus, the PBS-administered group was euthanized and the data after 12 days were excluded from the analysis.

### Lung tumor model

Next, the lung cancer cell line LLC1 was incubated in an incubator under a condition of 37°C and 5% CO₂ using DMEM medium composed of 10% fetal bovine serum, 2 mM L-glutamine, and 1% antibiotics-antimycotics (GIBCO, Cat No. 15240-062). Three passages were performed, and then the cells were harvested when the cells occupied 70% to 80% of the culture plate during the last passage. The supernatant of the harvested cells was removed, and an excipient (DMEM) was added thereto so that the cells are prepared to have a concentration of 1×10⁷/mL. A cell suspension containing the 1×10⁶ cells thus prepared was injected subcutaneously in the right flank of C57BL/6N mice (C57BL/6NHsd; KOATECH, Korea) to prepare a mouse lung tumor model. After one week, when the tumor volume grew to about 70 to 100 mm³, the prepared mouse model was divided into groups to be administered with PBS, IHD-W-VV01, and IHD-W-VV06 with 6 mice per group. Then, 50 ul of PBS (Welgene, Cat No. LB001-02) or each of the viruses at 1×10⁶ TCID₅₀/50 ul was administered once into the tumor. The results obtained by measuring the tumor volume after the virus administration are illustrated in Fig. 34.

As illustrated in Fig. 34, the results obtained by measuring the tumor size on day 14 after the virus treatment showed that the IHD-W-VV01-administered group and the IHD-W-VV06-administered group exhibit a smaller tumor volume than that of the PBS-administered group. It was found that when the tumor volume is compared among the virus-administered groups, the IHD-W-VV06-administered group exhibits a smaller tumor volume than that of the IHD-W-VV01-administered group.

### Experimental Example 2.2. Determination of tumor suppression efficacy, in animal model, of WR-VV01 and WR-VV06 viruses

Comparison of anti-tumor effects between the recombinant WR-VV01 and WR-VV06 as produced in Example 2 was made in a mouse model.

### Lung tumor model

Next, the lung cancer cell line LLC1 was incubated in an incubator under a condition of 37°C and 5% CO₂ using DMEM medium composed of 10% fetal bovine serum, 2 mM L-glutamine, and 1% antibiotics-antimycotics (GIBCO, Cat No. 15240-062). Three passages were performed, and then the cells were harvested when the cells occupied 70% to 80% of the culture plate during the last passage. The supernatant of the harvested cells was removed, and an excipient (DMEM) was added thereto so that the cells are prepared to have a concentration of 1×10⁷/mL. A cell suspension containing the 1×10⁶ cells thus prepared was injected subcutaneously in the right flank of C57BL/6N mice (C57BL/6NHsd; KOATECH, Korea) to prepare a mouse lung tumor model. After one week, when the tumor volume grew to about 70 to 100 mm³, the prepared mouse model was divided into groups to be administered with PBS, WR-VV01, and WR-VV06 with 6 mice per group. Then, 50 ul of PBS (Welgene, Cat No. LB001-02) or each of the viruses at 1×10⁶ TCID₅₀/50 ul was administered once into the tumor. The results obtained by measuring the tumor volume after the virus administration are illustrated in Fig. 35.

As illustrated in Fig. 35, the results obtained by measuring the tumor size on day 14 after the virus treatment showed that the WR-VV01-administered group and the WR-VV06-administered group exhibit a smaller tumor volume than that of the PBS-administered group. It was found that when the tumor volume is compared among the virus-administered groups, the WR-VV06-administered group exhibits a smaller tumor volume than that of the WR-VV01-administered group.

### Experimental Example 2.3. Determination of tumor suppression efficacy, in animal model, of Lister-VV01 and Lister-VV06 viruses

Comparison of anti-tumor effects between the recombinant Lister-VV01 and Lister-VV06 as produced in Example 3 was made in a mouse model.

### Lung tumor model

Next, the lung cancer cell line LLC1 was incubated in an incubator under a condition of 37°C and 5% CO₂ using DMEM medium composed of 10% fetal bovine serum, 2 mM L-glutamine, and 1% antibiotics-antimycotics (GIBCO, Cat No. 15240-062). Three passages were performed, and then the cells were harvested in a case where the cells occupied 70% to 80% of the culture plate during the last passage. The supernatant of the harvested cells was removed, and an excipient (DMEM) was added thereto so that the cells are prepared to have a concentration of 1×10⁷/mL. A cell suspension containing the 1×10⁶ cells thus prepared was injected subcutaneously in the right flank of C57BL/6N mice (C57BL/6NHsd; KOATECH, Korea) to prepare a mouse lung tumor model. After one week, when the tumor volume grew to about 70 to 100 mm³, the prepared mouse model was divided into groups to be administered with PBS, Lister-VV01, and Lister-VV06 with 6 mice per group. Then, 50 ul of PBS (Welgene, Cat No. LB001-02) or each of the viruses at 1×10⁶ TCID₅₀/50 ul was administered once into the tumor. The results obtained by measuring the tumor volume after the virus administration are illustrated in Fig. 36.

As illustrated in Fig. 36, the results obtained by measuring the tumor size on day 14 after the virus treatment showed that the Lister-VV06-administered group exhibits a smaller tumor volume than that of the PBS-administered group. It was found that when the tumor volume is compared among the virus-administered groups, the Lister-VV06-administered group exhibits a smaller tumor volume than that of the Lister-VV01-administered group.

### III. Determination of in vitro cell-killing ability of recombinant vaccinia virus in which VGF, TK, and K3L genes are deleted or expression thereof is inactivated and expression of sPD1-Fc, PH20, and IL-12 genes is induced

### Experimental Example 1. Determination of tumor cell-killing efficacy caused by administration of IHD-W-VV06, WR-VV06, or Lister-VV06

CCK-8 assay was performed to identify whether the recombinant vaccinia virus IHD-W-VV06 as produced in Example 1, the recombinant vaccinia virus WR-VV06 as produced in Example 2, or the recombinant vaccinia virus Lister-VV06 as produced in Example 3 exhibits killing ability against various types of cancer cells.

In order to identify cell-killing ability of the recombinant virus in various mouse cancer cell lines, a cancer cell line was prepared as shown in Table 24 below, and incubated in an incubator under a condition of 37°C and 5% CO₂. Then, the cell line was dispensed into a 96-well plate.

**[Table 24]**

| Carcinoma | Cell line | Medium used (Culture medium: containing 10% fetal bovine serum) |
|---|---|---|
| Colorectal cancer | CT26-WT | RPMI medium containing 2% fetal bovine serum and 2 mM L-glutamine |
| Renal cancer | Renca | RPMI medium containing 2% fetal bovine serum and 2 mM L-glutamine |
| Liver cancer | Hep-55.1C | RPMI medium containing 2% fetal bovine serum and 4 mM L-glutamine |
| Lung cancer | LLC1 | DMEM medium containing 2% fetal bovine serum and 2 mM L-glutamine |
| Breast cancer | JC | RPMI medium containing 2% fetal bovine serum and 2 mM L-glutamine |
| Breast cancer | 4T1 | RPMI medium containing 2% fetal bovine serum and 2 mM L-glutamine |
| Rectal cancer | CMT93 | RPMI medium containing 2% fetal bovine serum and 4 mM L-glutamine |
| Melanoma | B16F10 | RPMI medium containing 2% fetal bovine serum and 4 mM L-glutamine |

Here, the respective cell lines were dispensed such that the number of cells per well is 2 × 10⁴ cells for CT26-WT and Hep-55.1C, 1 × 10⁴ cells for Renca, LLC1, 4T1, CMT93, and B16F10, and 2 × 10³ cells for JC. After 24 hours of incubation, given the cell-killing ability of the wild-type virus of each of the recombinant viruses, the cell lines were infected with the recombinant vaccinia viruses as follows: to 1 MOI for IHD-W-VV06, to 1 MOI for WR-VV06, and to 10 MOI for Lister-VV06. Here, no-virus-treated cells were used as respective control groups. After 3 days, the cells were stained with CCK-8 (Dojindo, Cat No. CK04) solution to determine the cell viability of the cancer cell lines. The results are illustrated in Fig. 37.

As illustrated in Fig. 37, regardless of the vaccinia strain, recombinant vaccinia viruses in which VGF, TK, and K3L genes are deleted or expression thereof is inactivated and expression of sPD1-Fc, PH20, and IL-12 genes is induced exhibit killing ability against various types of cancer cells.

## Claims

1. A recombinant vaccinia virus, comprising:
a gene encoding soluble programmed cell death protein-1 (sPD-1), a gene encoding hyaluronidase, and a gene encoding interleukin (IL)-12.

2. The recombinant vaccinia virus of claim 1, wherein the sPD-1 comprises the amino acid sequence of SEQ ID NO: 131 or SEQ ID NO: 133.

3. The recombinant vaccinia virus of claim 1, wherein the gene encoding sPD-1 comprises the nucleotide sequence of SEQ ID NO: 132 or SEQ ID NO: 134.

4. The recombinant vaccinia virus of claim 1, wherein the hyaluronidase comprises the amino acid sequence of SEQ ID NO: 135.

5. The recombinant vaccinia virus of claim 1, wherein the gene encoding hyaluronidase comprises the nucleotide sequence of SEQ ID NO: 136.

6. The recombinant vaccinia virus of claim 1, wherein the IL-12 comprises the amino acid sequence of SEQ ID NO: 137.

7. The recombinant vaccinia virus of claim 1, wherein the gene encoding IL-12 comprises any one nucleotide sequence selected from SEQ ID NO: 138, 139, and 140.

8. The recombinant vaccinia virus of claim 1, wherein the vaccinia virus has suppressed expression of any one selected from the group consisting of a gene encoding K3L, a gene encoding thymidine kinase (TK), a gene encoding vaccinia growth factor (VGF), and combinations thereof.

9. The recombinant vaccinia virus of claim 1, wherein the vaccinia virus is of a strain selected from the group consisting of Western Reserve (WR), New York Vaccinia Virus (NYVAC), Wyeth (The New York City Board of Health), LC16m8, Lister, Copenhagen, Tian Tan, USSR, Tashkent, Evans, International Health Division-J (IHD-J), International Health Division-White (IHD-W), variants thereof, and combinations thereof.

10. The recombinant vaccinia virus of claim 1, wherein the vaccinia virus strain is IHD-W.

11. A composition that contains, as an active ingredient, the recombinant vaccinia virus of any one of claims 1 to 10 for use in preventing or treating cancer.

12. The composition for use according to claim 11, wherein the cancer is solid cancer or blood cancer, optionally:
i) wherein the solid cancer is any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, and combinations thereof; or
ii) wherein the blood cancer is any one selected from the group consisting of lymphoma, acute leukemia, multiple myeloma, and combinations thereof.

## Patentansprüche

1. Ein rekombinantes Vaccinia Virus, umfassend:
ein Gen, das für soluble Programmed Cell Death protein-1 (sPD-1) kodiert, ein Gen, das für Hyaluronidase kodiert und ein Gen, das für Interleukin (IL)-12 kodiert.

2. Das rekombinante Vaccinia Virus nach Anspruch 1, wobei das sPD-1 die Aminosäuresequenz von SEQ DI NO: 131 oder SEQ ID NO: 133 umfasst.

3. Das rekombinante Vaccinia Virus nach Anspruch 1, wobei das Gen, das für sPD-1 kodiert die Nukleotidsequenz von SEQ ID NO: 132 oder SEQ ID NO: 134 umfasst.

4. Das rekombinante Vaccinia Virus nach Anspruch 1, wobei die Hyaluronidase die Aminosäuresequenz von SEQ ID NO. 135 umfasst.

5. Das rekombinante Vaccinia Virus nach Anspruch 1, wobei das Gen, das für die Hyaluronidase kodiert, die Nukleotidsequenz von SEQ ID NO: 136 umfasst.

6. Das rekombinante Vaccinia Virus nach Anspruch 1, wobei das IL-12 die Aminosäuresequenz von SEQ ID NO. 137 umfasst.

7. Das rekombinante Vaccinia Virus nach Anspruch 1, wobei das Gen, das für IL-12 kodiert, eine der Nukleotidsequenzen ausgewählt aus SEQ ID NO: 138, 139 und 140 umfasst.

8. Das rekombinante Vaccinia Virus nach Anspruch 1, wobei das Vaccinia Virus eine unterdrückte Expression eines ausgewählt aus der Gruppe bestehend aus einem Gen, das K3L kodiert, einem Gen, das die Thymdinkinase (TK) kodiert, einem Gen, das den Vaccina Wachstumsfaktor (VGF) kodiert und Kombinationen davon umfasst.

9. Das rekombinante Vaccinia Virus nach Anspruch 1, wobei das Vaccinia Virus aus einem Stamm ist, der ausgewählt ist aus der Gruppe bestehend aus Western Reserve (WR), New York Vaccinia Virus (NYVAC), Wyeth (The New York City Board of Health), LC16m8, Lister, Copenhagen, Tian Tan, USSR, Tashkent, Evans, International Health Division-J (IHD-J), International Health Division-White (IHD-W), Varianten davon und Kombinationen davon.

10. Das rekombinante Vaccinia Virus nach Anspruch 1, wobei der Vaccinia Virus Stamm IHD-W ist.

11. Eine Zusammensetzung, die als aktiven Zusatzstoff, das rekombinante Vaccinia Virus nach einem der Ansprüche 1 bis 10 enthält, zur Verwendung bei der Prävention oder Behandlung von Krebs.

12. Die Zusammensetzung zur Verwendung nach Anspruch 11, wobei der Krebs ein solider Krebs oder Blutkrebs ist, optional:
i) wobei der solide Krebs einer ausgewählt aus der Gruppe bestehend aus Lungenkrebs, kolorektalem Krebs, Prostatakrebs, Schilddrüsenkrebs, Brustkrebs, Hirnkrebs, Kopf- und Halskrebs, Speiseröhrenkrebs, Hautkrebs, Thymuskrebs, Magenkrebs, Darmkrebs, Leberkrebs, Eierstockkrebs, Gebärmutterkrebs, Blasenkrebs, Rektalkrebs, Gallenblasenkrebs, Gallenwegskrebs, Bauchspeicheldrüsenkrebs und Kombinationen davon ist; oder
ii) wobei der Blutkrebs einer ausgewählt aus der Gruppe bestehend aus Lymphom, akuter Leukämie, multiplem Myelom und Kombinationen davon ist.

## Revendications

1. - Virus de la vaccine recombinant, comprenant : un gène codant pour la protéine 1 de mort cellulaire programmée soluble (sPD-1), un gène codant pour la hyaluronidase et un gène codant pour l'interleukine (IL)-12.

2. - Virus de la vaccine recombinant selon la revendication 1, dans lequel la sPD-1 comprend la séquence d'acides aminés de SEQ ID NO : 131 ou SEQ ID NO : 133.

3. - Virus de la vaccine recombinant selon la revendication 1, dans lequel le gène codant pour sPD-1 comprend la séquence nucléotidique de SEQ ID NO : 132 ou SEQ ID NO : 134.

4. - Virus de la vaccine recombinant selon la revendication 1, dans lequel la hyaluronidase comprend la séquence d'acides aminés de SEQ ID NO : 135.

5. - Virus de la vaccine recombinant selon la revendication 1, dans lequel le gène codant pour la hyaluronidase comprend la séquence nucléotidique de SEQ ID NO : 136.

6. - Virus de la vaccine recombinant selon la revendication 1, dans lequel l'IL-12 comprend la séquence d'acides aminés de SEQ ID NO : 137.

7. - Virus de la vaccine recombinant selon la revendication 1, dans lequel le gène codant pour l'IL-12 comprend l'une quelconque des séquences nucléotidiques choisies parmi SEQ ID NO : 138, 139 et 140.

8. - Virus de la vaccine recombinant selon la revendication 1, dans lequel le virus de la vaccine présente une suppression de l'expression de l'un quelconque choisi dans le groupe constitué d'un gène codant pour K3L, d'un gène codant pour la thymidine kinase (TK), d'un gène codant pour le facteur de croissance de la vaccine (VGF), et des combinaisons de ceux-ci.

9. - Virus de la vaccine recombinant selon la revendication 1, dans lequel le virus de la vaccine provient d'une souche choisie dans le groupe constitué par Western Reserve / Réserve de l'Ouest (WR), New York Vaccinia Virus / Virus de la Vaccine de New York (NYVAC), Wyeth (The New York City Board of Health / Conseil de la Santé de la ville de New York), LC16m8, Lister, Copenhagen, Tian Tan, USSR, Tachkent, Evans, International Health Division-J (IHD-J), International Health Division-White (IHD-W), les variantes de celles-ci et les combinaisons de celles-ci.

10. - Virus de la vaccine recombinant selon la revendication 1, dans lequel la souche de virus de la vaccine est IHD-W.

11. - Composition comprenant, en tant que principe actif, le virus de la vaccine recombinant selon l'une quelconque des revendications 1 à 10 pour une utilisation dans la prévention ou le traitement du cancer.

12. - Composition pour l'utilisation selon la revendication 11, dans laquelle le cancer est un cancer solide ou un cancer du sang, facultativement :
i) dans laquelle le cancer solide est l'un quelconque choisi dans le groupe constitué par le cancer du poumon, le cancer colorectal, le cancer de la prostate, le cancer de la thyroïde, le cancer du sein, le cancer du cerveau, le cancer de la tête et du cou, le cancer de l'œsophage, le cancer de la peau, le cancer du thymus, le cancer de l'estomac, le cancer du côlon, le cancer du foie, le cancer de l'ovaire, le cancer de l'utérus, le cancer de la vessie, le cancer rectal, le cancer de la vésicule biliaire, le cancer des voies biliaires, le cancer du pancréas et des combinaisons de ceux-ci ; ou
ii) dans laquelle le cancer du sang est l'un quelconque choisi dans le groupe constitué par le lymphome, la leucémie aiguë, le myélome multiple et des combinaisons de ceux-ci.
